# EUROPÄISCHE PATENTSCHRIFT

(11) **EP 1 272 223 B1**
(45) Veröffentlichungstag und Bekanntmachung des Hinweises auf die Patenterteilung: **31.05.2006**
(21) Anmeldenummer: 01933693.2
(22) Anmeldetag: 13.03.2001
(51) Int. Cl.: A61K 47/48, A61P 37/00, A61P 35/00, A61P 31/00, A61P 29/00

(54) **THERAPEUTISCHE UND DIAGNOSTISCHE LIGANDENSYSTEME MIT TRANSPORTMOLEKÜLBINDENDEN EIGENSCHAFTEN UND DIESE ENTHALTENDE ARZNEIMITTEL**
THERAPEUTIC AND DIAGNOSTIC LIGAND SYSTEMS COMPRISING TRANSPORT MOLECULE BINDING PROPERTIES AND MEDICAMENTS CONTAINING THE SAME
SYSTEMES DE LIGANDS THERAPEUTIQUES ET DIAGNOSTIQUES POUVANT LIER UNE MOLECULE PORTEUSE ET MEDICAMENTS CONTENANT CES LIGANDS

(30) Priorität: 13.03.2000 DE 10012120
(43) Veröffentlichungstag der Anmeldung: 08.01.2003
(73) Patentinhaber: KTB Tumorforschungsgesellschaft mbH, 79106 Freiburg (DE)
(72) Erfinder: KRATZ, Felix, 79241 Ihringen (DE)
(74) Vertreter: Hock, Joachim
(86) Internationale Anmeldenummer: PCT/EP2001/002833
(87) Internationale Veröffentlichungsnummer: WO 2001/068142

(56) Entgegenhaltungen:
- EP-A- 0 624 377
- WO-A-00/02050
- WO-A-91/05806
- WO-A-96/39183
- WO-A-98/08859
- US-A- 5 612 474
- US-A- 5 919 815
- TAKAHASHI T ET AL: "Design and synthesis of a water-soluble taxol analogue: taxol-sialyl conjugate" BIOORGANIC & MEDICINAL CHEMISTRY LETTERS, OXFORD, GB, Bd. 8, Nr. 1-6, 6. Januar 1998 (1998-01-06), Seiten 113-116, XP004136633 ISSN: 0960-894X

## Beschreibung

Die vorliegende Erfindung betrifft Trägermolekül-bindende Ligandenverbindungen, die eine therapeutisch und/oder diagnostisch wirksame Substanz und eine Trägermolekül-affine Substanz mit einer hohen Assoziationskonstante zu dem Trägermolekül umfassen, sowie diese Ligandenverbindungen enthaltende Arzneimittel und diagnostische Kits.

In der Blutbahn zirkulieren eine Reihe von Transportproteinen für Lipide, Hormone, Metaboliten, Pharmaka und Vitamine. Beispiele solcher Transportproteine sind Albumin, Haptoglobin, Präalbumin, Low-Density Lipoprotein (LDL), Retinol-bindendes Protein, Transcortin, Vitamin D-bindendes Protein bzw. Transcobalamin. Die Affinität zwischen dem Transportprotein und seinem Liganden wird insbesondere durch die Assoziationskonstante K_{A} beschrieben.

Es ist bekannt, daß eine Reihe von Verbindungen, wie z.B. Farbstoffe, Porphyrine, organische Säuren oder Pharmaka, mit bestimmten Transportproteinen in der Blutbahn eine ausgeprägte nicht-kovalente, d.h. physikalische Wechselwirkung, eingehen, wobei eine Selektivität für bestimmte Transportproteine, beispielsweise für Albumin, vorhanden ist.

Von mehreren Blutproteinen ist weiterhin bekannt, daß sie sich im pathogenen Gewebe, z.B. in malignem oder entzündetem Gewebe, anreichem. Eine solche Anreicherung wurde beispielsweise für das Serumprotein Albumin nachgewiesen (Kratz, F., Beyer U. (1998) Serum proteins as drug carriers of anticancer agents, a review. *Drug Delivery, 5*, 1-19; siehe auch Kratz, F.; Beyer, U ; Schütte , M.T. (1999) Drug Polymer Conjugates Containing Acid-Cleavable Bonds Critical Reviews in Therapeutic Drug Carrier System 16(3):245-287).

Der vorliegenden Erfindung liegt somit die Aufgabe zugrunde, die vorstehend genannten Trägermolekül-bindenden Eigenschaften, wie beispielsweise die Bindung an Transportproteine in der Blutbahn aufgrund ausgeprägter physikalischer Wechselwirkungen, therapeutisch und diagnostisch zu nutzen und ein neues Ligandensystem mit Trägermolekül-bindenden Eigenschaften und diese Trägermolekül-bindenden Verbindungen enthaltende Arzneimittel bereitzustellen.

Diese Aufgabe wird durch die in den Ansprüchen gekennzeichneten Ausführungsformen der vorliegenden Erfindung gelöst.

Insbesondere wird eine Trägermolekül-'bindende Ligandenverbindurig bereitgestellt, umfassend mindestens eine therapeutisch bzw. pharmazeutisch und/oder diagnostisch wirksame Substanz und mindestens eine Trägermolekül-affine Substanz, die eine Assoziations- bzw. Bindungskonstante K_{A} zu dem Trägermolekül von > 10³ M⁻¹, bevorzugt > 10⁵ M⁻¹, noch bevorzugter > 10⁷ M⁻¹, am meisten bevorzugt > 10⁸ M⁻¹, über eine nicht-kovalente Bindung aufweist.

Der Ausdruck "therapeutisch bzw. pharmazeutisch wirksame Substanz" bedeutet, daß die betreffende Substanz entweder selbst oder nach ihrer Umsetzung durch den Stoffwechsel im jeweiligen Organismus eine pharmakologische Wirkung hervorruft und umfaßt somit auch die sich durch diese Umsetzungen ergebenden Derivate. Selbstverständlich kann die therapeutisch wirksame Substanz ein einzelnes (beispielsweise nur als Zytostatikum) oder ein breites (beispielsweise als Zytostatikum und als Antirheumatikum usw.) pharmakologisches Wirkspektrum aufweisen. Der Ausdruck "diagnostisch wirksame Substanz" bedeutet, daß die betreffende Substanz mittels geeigneter chemischer und/oder physikalischer Meßmethoden im Organismus oder Teilen davon wie beispielsweise Zellen und/oder Flüssigkeiten wie beispielsweise dem Serum nachweisbar, vorzugsweise auch quantifizierbar ist.

Die Trägermolekül-affine Substanz in der erfindungsgemäßen Trägermolekül-bindenden Ligandenverbindung weist keine kovalente Wechselwirkung mit dem Trägermolekül auf, d.h. die Trägermolekül-affine Substanz bindet aufgrund von Wechselwirkungen physikalischer Natur wie elektrostatischer Wechselwirkungen, Wasserstoffbrückenbindungen, van-der-Waals-Bindungen, und/oder hydrophoben Wechselwirkungen an das Trägermolekül. Dabei ist es hinsichtlich der Spezifität der Trägermolekül-affinen Substanz zum Trägermolekül entscheidend, daß die Gleichgewichtskonstante der Assoziationsreaktion zwischen dem Trägermolekül und der Trägermolekül-affinen Substanz ausreichend groß ist, was erfindungsgemäß einem Wert von > 10³ M⁻¹ (log K_{A} > 3), bevorzugt mindestens 10⁴ M⁻¹ (log K_{A} mindestens 4), mehr bevorzugt > 10⁵ M⁻¹ (log K_{A} > 5), noch bevorzugter > 10⁷ M⁻¹ (log K_{A} > 7) und am meisten bevorzugt > 10⁸ M⁻¹ (log K_{A} > 8) entspricht In der erfindungsgemäßen Ligandenverbindung kann die therapeutisch und/oder diagnostisch wirksame Substanz direkt mit der Trägermolekül-affinen Substanz verbunden sein. Gemäß einer bevorzugten Ausführungsform der erfindungsgemäßen Ligandenverbindung ist die Verknüpfung zwischen der therapeutisch und/oder diagnostisch wirksamen Substanz und der Trägermolekül-affinen Substanz entweder im Körper nicht spaltbar oder diese Verknüpfung ist pH-abhängig und/oder enzymatisch spaltbar.

Gemäß einer weiteren bevorzugten Ausführungsform der erfindungsgemäßen Ligandenverbindung sind die therapeutisch und/oder diagnostisch wirksame Substanz und die Trägermolekül-affine Substanz über ein Spacermolekül miteinander verbunden. Dabei kann, wie schon vorstehend für die direkte Verknüpfung zwischen therapeutisch und/oder diagnostisch wirksamer Substanz und Trägermolekül-affiner Substanz ausgeführt, das Spacermolekül und/oder die Verknüpfung zwischen der therapeutisch und/oder diagnostisch wirksamen Substanz und dem Spacermolekül und/oder die Verknüpfung der Trägermolekül-affinen Substanz und dem Spacermolekül entweder im Körper nicht spaltbar sein oder das Spacermolekül und/oder die genannten Verknüpfungen können pH-abhängig und/oder enzymatisch spaltbar sein.

Gemäß einer weiteren bevorzugten Ausführungsform enthält die direkte Verknüpfung zwischen der therapeutisch und/oder diagnostisch wirksamen Substanz und der Trägermolekül-affinen Substanz oder das Spacermolekül und/oder die Verknüpfung zwischen der therapeutisch und/oder diagnostisch wirksamen Substanz und dem Spacermolekül und/oder die Verknüpfung zwischen der Trägermolekül-affinen Substanz und dem Spacermolekül mindestens eine säurelabile Bindung. Beispiele solcher säurelabiler Bindungen sind Ester-, Acetal-, Ketal-, Imin-, Hydrazon, Carboxylhydrazon- oder Sulfonylhydrazonverbindungen.

Gemäß einer weiteren Ausführungsform der erfindungsgemäßen Ligandenverbindung enthält entweder die direkte Verknüpfung zwischen der therapeutisch und/oder diagnostisch wirksamen Substanz und der Trägermolekül-affinen Substanz oder das Spacermolekül und/oder die Verknüpfung zwischen der therapeutisch und/oder diagnostisch wirksamen Substanz und dem Spacermolekül und/oder die Verknüpfung zwischen der Trägermolekül-affinen Substanz und dem Spacermolekül mindestens eine Peptidbindung. Vorzugsweise liegt die Peptidbindung innerhalb einer Peptidsequenz vor, welche mindestens eine Spaltsequenz einer Protease enthält. Daher kann die mindestens eine Peptidbindung durch Anfügen einer Peptidsequenz in die direkte Verknüpfung zwischen der therapeutisch und/oder diagnostisch wirksamen Substanz und der Trägermolekül-affinen Substanz oder in das Spacermolekül und/oder in die Verknüpfung zwischen der therapeutisch und/oder diagnostisch wirksamen Substanz und dem Spacermolekül und/oder in die Verknüpfung zwischen der Trägermolekül-affinen Substanz und dem Spacermolekül realisiert werden, d.h. die jeweilige Verknüpfung ist eine Peptidbindung, und besteht vorzugsweise aus etwa 1 bis 30 Aminosäuren. Die Peptidsequenz ist vorzugsweise auf die Substratspezifität bestimmter körpereigener Enzyme oder von Enzymen zugeschnitten, die in Mikroorganismen vorkommen bzw. von diesen gebildet werden. Dadurch wird die Peptidsequenz oder ein Teil dieser Sequenz im Körper von den Enzymen erkannt und das Peptid gespalten.

Die Enzyme sind beispielsweise Proteasen und Peptidasen, z.B. Matrix-Metalloproteasen (MMP1-20), Cysteinproteasen (z.B. Cathepsin B, D, L, H), Aspartylproteasen (z.B. Cathepsin D), Serinproteasen (z.B. Plasmin, "tissue-type" Plasminigen-Aktivator (tPA), "urokinase-type" Plasminogen-Aktivator), Kallikrein oder Kallikrein ähnliche Proteasen (z.B. Prostata-spezifisches Antigen), die bei Erkrankungen wie rheumatoider Arthritis oder Krebs verstärkt gebildet oder aktiviert sind, was zum exzessiven. Gewebeabbau, zu Entzündungen und zur Metastasierung führt. Target-Enzyme sind insbesondere MMP-2, MMP-3 und MMP-9, die Cathepsine B, D, H und L, tissue-type Plasminigen-Aktivator (tPA) und urokinase-type Plasminogen-Aktivator sowie Prostata-spezifisches Antigen, die als Schlüsselenzyme bei entzündlichen und malignen Erkrankungen identifiziert worden sind und als Proteasen bei den genannten pathologischen Prozessen beteiligt sind (Vassalli, J., Pepper, M.S. (1994), *Nature 370,* 14-15; Brown, P.D. 1995), *Advan. Enzyme Regul. 35*, 291-301; Schmitt M., et al., *J.Obsetrics&Gynaecology, 21,* 151-65,1995, T. T. Lah et al. (1998), *Biol. Chem. 379*, 125-301).

Gemäß einer weiteren Ausführungsform der erfindungsgemäßen Ligandenverbindung enthält entweder die direkte Verknüpfung zwischen der therapeutisch und/oder diagnostisch wirksamen Substanz und der Trägermolekül-affinen Substanz oder das Spacermolekül und/oder die Verknüpfung zwischen der therapeutisch und/oder diagnostisch wirksamen Substanz und dem Spacermolekül und/oder die Verknüpfung zwischen der Trägermolekül-affinen Substanz und dem Spacermolekül mindestens eine Bindung, die enzymatisch spaltbar ist, aber nicht aus einer Peptidbindung besteht. Beispiele sind Carbamatbindungen, bei denen durch Spaltung mit krankheitsspezifischen Enzymen, z.B. Glutathion-S-Transferasen, Glucuronidasen, Galactosidasen, der Wirkstoff oder ein Wirkstoffderivat freigesetzt wird.

Alle drei Bindungstypen - säurelabile Bindung, Peptidbindung, enzymatisch spaltbare Bindung, die keine Peptidbindung enthält - gewährleisten, daß die therapeutisch und/oder diagnostisch wirksame Substanz oder ein entsprechend aktives Derivat am Wirkort extrazellulär und/oder intrazellulär gespalten wird und die Substanz seine pharmazeutische und/oder diagnostische Wirkung entfalten kann.

Der Ausdruck "Trägermolekül" umfaßt sowohl natürliche als auch synthetische Moleküle, die dazu geeignet sind, die erfindungsgemäße Ligandenverbindung beispielsweise in Körperflüssigkeiten wie Blutserum zu transportieren. Geeignete Transportmoleküle sind natürlich vorkommende oder synthetische Makromoleküle, beispielsweise Polyethylenglykol (PEG) oder Dextrane und biologische Makromoleküle wie Proteine. Bevorzugte, als Trägermolekül geeignete Proteine sind aus der Gruppe der Serumproteine ausgewählt. Ein Beispiel für ein bevorzugtes geeignetes Serumprotein ist Albumin. Weitere geeignete Transportproteine als Trägermoleküle sind Transferrin, Haptoglobin, Präalbumin, Low-Density Lipoprotein (LDL), Retinol-bindendes Protein, Transcortin sowie Vitamin D-bindendes Protein bzw. Transcobalamin.

Geeignete Transportmoleküle sind somit natürlich vorkommende oder synthetische Makromoleküle, beispielsweise Polysaccharide, Polypeptide, Polyalkohole, Polyamine, Dendrimere, Copolymere, Polyethylenglykol (PEG) oder funküonaüsierte Polyethylenglykole, und biologische Makromoleküle wie Proteine.

Gemäß einer bevorzugten Ausführungsform ist die therapeutisch und/oder diagnostisch wirksame Substanz ein Zytostatikum, ein Zytokin, ein Immunsuppressivum, ein Antirheumatikum, ein Antiphlogistikum, ein Antibiotikum; ein Analgetikum, ein Virostatikum oder ein Antimykotikum Besonders geeignete Zytostatika der Ligandenverbindung der vorliegenden Erfindung sind die N-Nitrosohamstoffe wie Nimustin, die Anthrazykline Doxorubicin, Daunorubicin, Epirubicin, Idarubicin, Mitoxantron und Ametantron sowie verwandte Derivate, die Alkylantien Chlorambucil, Bendamustin, Melphalan und Oxazaphosphorine sowie verwandte Derivate, die Antimetabolite, beispielsweise Purin- oder Pyrimidinantagonisten und Folsäureantagonisten, wie Methotrexat, 5-Fluorouracil, 5'-Desoxy-5-fluorouridin, Gemcitabine und Thioguanin sowie verwandte Derivate, die Taxane Paclitaxel und Docetaxel sowie verwandte Derivate, die Camptothecine Topotecan, Irinotecan, 9-Aminocamptothecin und Camptothecin sowie verwandte Derivate, die Podophyllotoxinderivate Etoposid, Teniposid und Mitopodozid sowie verwandte Derivate, die Vinca-Alkaloide Vinblastin, Vincristin, Vindesin und Vinorelbin sowie verwandte Derivate, Calicheamicine, Maytansinoide, Epithilone wie Epithilon A und B und verwandte Derivate und cis-konfigurierte Platin(II)-Komplexverbindungen der allgemeinen Formeln I bis XIV: 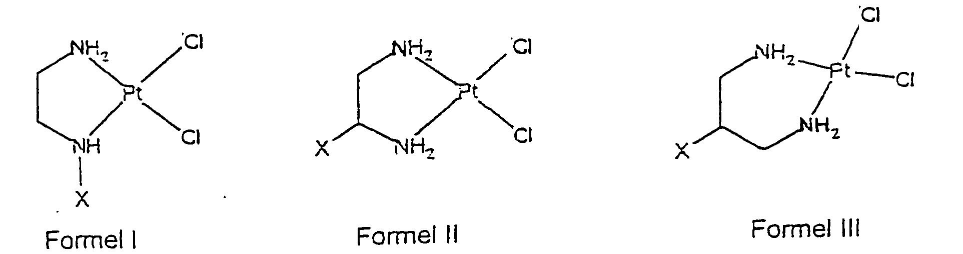 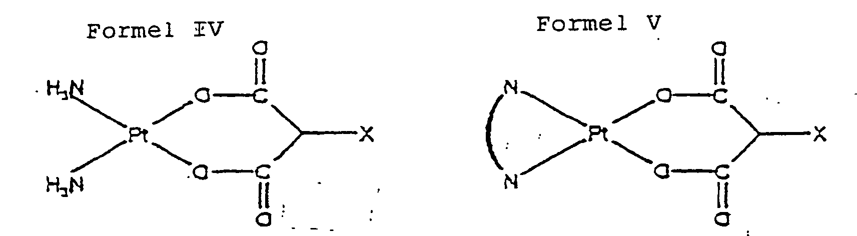 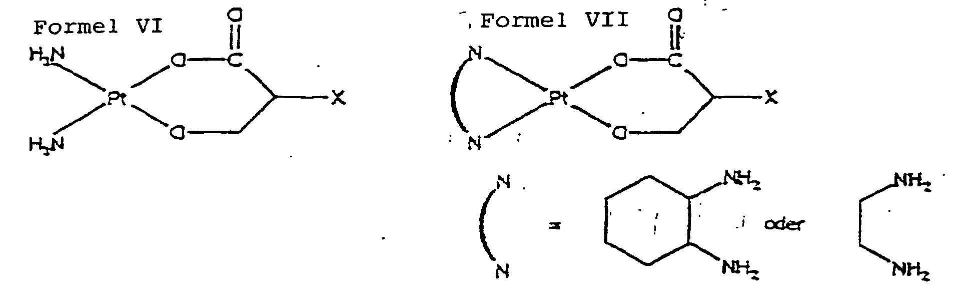 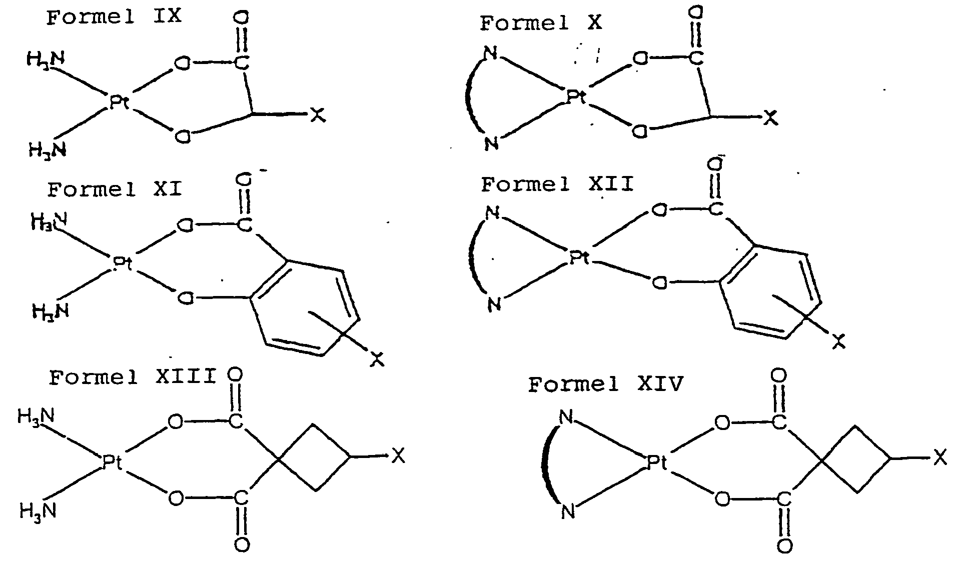 wobei X das Spacermolekül und/oder die Transportmolekül-affine Substanz ist.

Besonders geeignete Zytokine in Ligandenverbindungen der vorliegenden Erfindung sind beispielsweise Interleukin 2, Interferon α-2a, Interferon α-2b, Interferon β-1a, Interferon β-1b, Interferon γ-1b und verwandte Derivate. Die verwendeten Zytokine sind beispielsweise gentechnisch hergestellte Arzneimittel.

Besonders geeignete Immunsuppresiva in Ligandenverbindungen der vorliegenden Erfindung sind beispielsweise Cyclosporin A, FK 506 (Tacrolimus) und verwandte Derivate.

Besonders geeignete Antirheumatika in Konjugaten der vorliegenden Erfindung sind beispielsweise Methotrexat, Sulfasalazin, Chloroquin und verwandte Derivate.

Besonders geeignete Antiphlogistika und/oder Analgetika in Ligandenverbindungen der vorliegenden Erfindung sind beispielsweise Salicyisäurederivate, wie etwa Acetylsalicylsäure und verwandte Derivate, Pharmaka-Derivate, die eine Essig- oder Propionsäuregruppe aufweisen, wie etwa Diclofenac bzw. Indometacin oder Ibuprofen bzw. Naproxen, und Aminophenolderivate, wie etwa Paracetamol.

Besonders geeignete Antimykotika in Ligandenverbindungen der vorliegenden Erfindung sind beispielsweise Amphotericin B und verwandte Derivate.

Bevorzugte Virostatika in Ligandenverbindungen der vorliegenden Erfindung sind beispielsweise Nukleosidanaloga, wie Aciclovir, Ganciclovir, Idoxuridin, Ribavirin, Vidaribin, Zidovudin, Didanosin und 2',3'-Didesoxycytidin (ddC) und verwandte Derivate sowie Amantadin.

Bevorzugte Antibiotika in der erfindungsgemäßen Ligandenverbindung sind Sulfonamide, beispielsweise Sulanilamid, Sulfacarbamid und Sulfametoxydiazin und verwandte Derivate, Penicilline, beispielsweise 6-Aminopenicillansäure, Penicillin G sowie Penicillin V und verwandte Derivate, Isoxazoylpenicilline, wie Oxacillin, Cloxacillin und Flucloxacillin sowie verwandte Derivate, α-substituierte Benzylpenicilline, wie Ampicillin, Carbenicillin, Pivampicillin, Amoxicillin und verwandte Derivate, Acylaminopenicelline, beispielsweise Meztocillin, Azlocillin, Piperacillin, Apalicillin und verwandte Derviate, Amidinopenicilline, beispielsweise Mecillinam, atypische β-Lactame, wie Imipenam und Aztreonam, Cephalosporine, beispielsweise Cefalexin, Cefradin, Cefaclor, Cefadroxil, Cefixim, Cefpodoxim, Cefazolin, Cefazedon, Cefuroxim, Cefamandol, Cefotiam, Cefoxitin, Cefotetan, Cefmetazol, Latamoxef, Cefotaxim, Ceftriaxon, Ceftizoxim, Cefmonoxim, Ceftazidim, Cefsulodin und Cefoperazon sowie verwandte Derivate, Tetracycline, wie Tetracyclin, Chlortetracyclin, Oxytetracyclin, Demeclocyclin, Rolitetracyclin, Doxycyclin, Minocyclin und verwandte Derivate, Chloramphenicole, wie Chloramphenicol und Thiamphenicol sowie verwandte Derivate, Gyrasehemmstoffe, beispielsweise Nalixidinsäure, Pipemidsäure, Norfloxacin, Ofloxacin, Ciprofloxacin und Enoxacin sowie verwandte Derivate, und Tuberkulosemittel, wie Isoniazid und verwandte Derivate.

Selbstverständlich kann in der erfindungsgemäßen Ligandenverbindung pro Mol eine einzelne Therapeutikum- und/oder Diagnostikumspezies (beispielsweise ein Therapeutikum mit einem Zytostatikum als therapeutisch aktiver Substanz) oder unterschiedliche Therapeutikum- und/oder Diagnostikumspezies (beispielsweise mehrere unterschiedliche Zytostatika oder ein Zytostatikum und ein Antirheumatikum usw. als therapeutisch aktive Substanz) gebunden vorliegen.

Gemäß einer weiteren bevorzugten Ausführungsform der erfindungsgemäßen Ligandenverbindung umfaßt das Spacermolekül einen substituierten oder unsubstituierten, verzweigt- oder unverzweigtkettigen aliphatischen Alkylrest und/oder mindestens einen substituierten oder unsubstituierten Arylrest. Der aliphatische Alkylrest enthält vorzugsweise 1 bis 20 Kohlenstoffatome, die teilweise durch Sauerstoff- oder Stickstoffatome ersetzt sein können, um beispielsweise die Wasserlöslichkeit zu erhöhen, wobei sich solche Reste vorzugsweise von einer Oligoethylenoxid- oder -propylenoxidkette ableiten. Besonders geeignete Reste, die von Oligoethylenoxid- oder -propylenoxidketten abgeleitet sind, umfassen beispielsweise Diethylenglycol-. Triethylenglycol-, Tetraethylenglycol-, Pentaethylenglycol-, Hexaethylenglycol, Heptaethylenglycol- und Octaethylenglykolketten sowie die analogen Oligopropylenglycolketten. Ein bevorzugter Arylrest ist ein unsubstituierter oder substituierter Phenylrest, bei welchem ebenfalls ein oder mehrere Kohlenstoffatome durch Heteroatome ersetzt sein können. Bevorzugte Substituenten des aliphatischen Alkylrests bzw. des Arylrests sind hydrophile Gruppen, wie Sulfonsäure- (einschließlich ihrer Salze mit Alkali- bzw. Erdalkalimetallen), Carboxyl- (einschließlich ihrer Salze mit Alkali- bzw. Erdalkalimetallen), Amino-, Aminoalkyl- und Hydroxygruppen.

Die Wasserlöslichkeit der erfindungsgemäßen Ligandenverbindungen kann auch dadurch erreicht bzw. verbessert werden, daß die Trägermolekül-affine Substanz selbst eine oder mehrere wasserlöslichen Gruppen, etwa eine Sulfonsäure-(einschließlich ihrer Salze mit Alkali- bzw. Erdalkalimetallen), Carbonsäure-(einschließlich ihrer Salze mit Alkali- bzw. Erdalkalimetallen), Amino-, Aminoalkyl- und/oder Hydroxygruppen, aufweisen, oder daß solche durch synthetische Schritte eingeführt werden.

Bevorzugte diagnostisch wirksame Substanzen der erfindungsgemäßen Ligandenbindung enthalten beispielsweise ein oder mehrere Radionuklide, ein oder mehrere Radionuklide umfassende, vorzugsweise solche Radionuklide komplexierende Liganden, ein oder mehrere Positronenstrahler, ein oder mehrere NMR-Kontrastmittel, eine oder mehrere fluoreszierende . Verbindung(en) oder ein oder mehrere Kontrastmittel im nahen IR-Bereich.

Die erfindungsgemäße Ligandenverbindung, enthaltend mindestens eine therapeutisch und/oder diagnostisch wirksame Substanz (WS), mindestens eine Trägermolekül-affine Substanz (TAS) und gegebenenfalls ein Spacermolekül (SM), kann je nach der vorliegenden funktionellen Gruppe gemäß einer der folgenden allgemeinen Beschreibungen hergestellt werden.

Therapeutisch und/oder diagnostisch wirksame Substanzen der erfindungsgemäßen Ligandenverbindungen, die eine HOOC-Gruppe besitzen, können beispielsweise folgendermaßen derivatisiert werden:

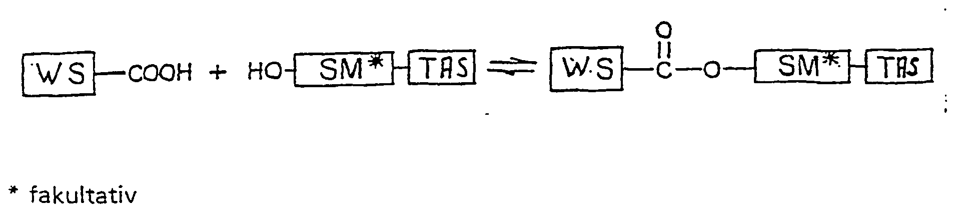

Die Veresterung erfolgt dabei durch im Stand der Technik bekannte Verfahren.

Es ist weiterhin möglich, die HOOC-Gruppe in eine Hydrazidgruppe zu überführen, z.B. durch Umsetzen mit tert.-Alkylcarbazaten und anschließende Spaltung mit Säuren (vgl. DE-A-196 36 889), und die eine Hydrazidgruppe aufweisende Verbindung mit einer eine Carbonylkomponente enthaltenden Gruppe, bestehend aus der Transportmolekül-affinen Substanz und dem Spacermolekül, umzusetzen, wie u.a. in DE-A-196 36 889 beschrieben ist:

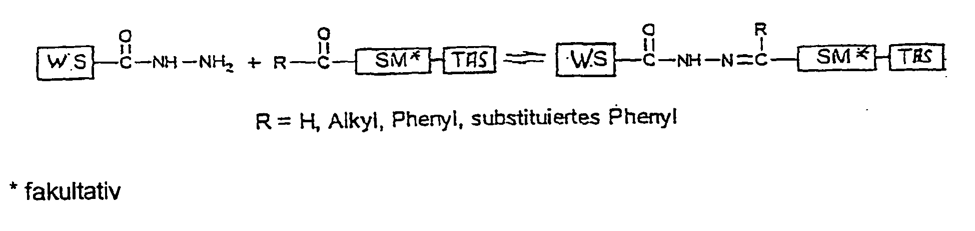

Therapeutisch und/oder diagnostisch wirksame Substanzen der erfindungsgemäßen Ligandenverbindungen, die eine H₂N-Gruppe besitzen, können beispielsweise folgendermaßen derivatisiert werden:

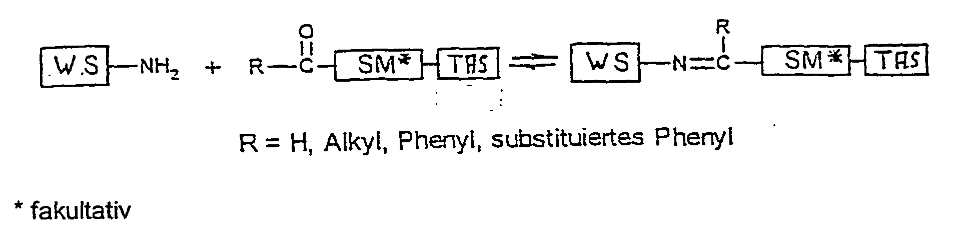

Die Reaktion zu den Iminderivaten erfolgt dabei durch im Stand der Technik bekannte Verfahren.

Therapeutisch und/oder diagnostisch wirksame Substanzen der erfindungsgemäßen Ligandenverbindungen, die eine HO-Gruppe besitzen, können beispielsweise folgendermaßen derivatisiert werden:

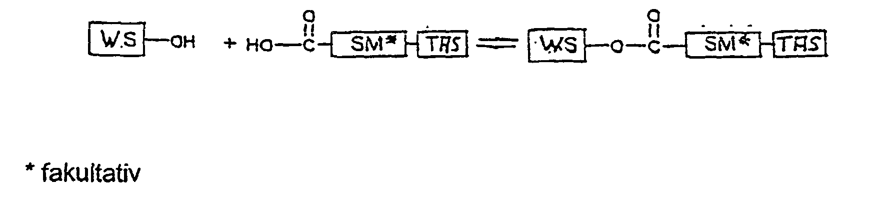

Die Veresterung erfolgt dabei durch im Stand der Technik bekannte Verfahren.

Therapeutisch und/oder diagnostisch wirksame Substanzen der erfindungsgemäßen Ligandenverbindungen, die eine Carbonylkomponente besitzen, können beispielsweise folgendermaßen derivatisiert werden:

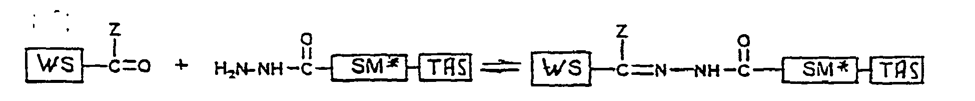

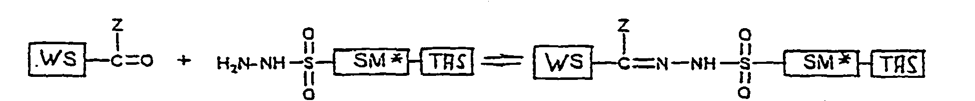

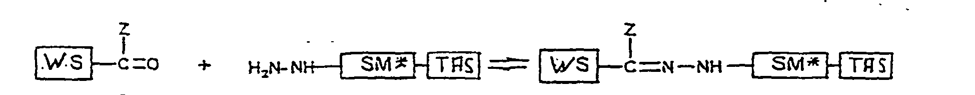

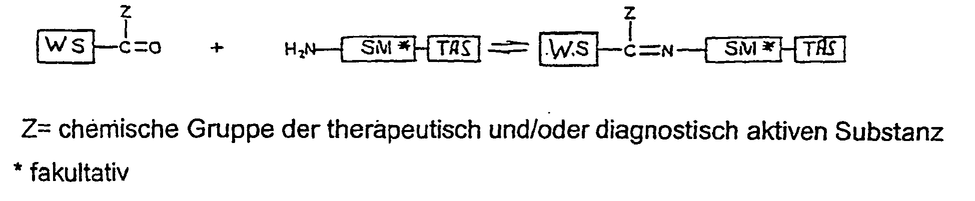

Die Umsetzung zu den Carboxyhydrazon-, Sulfonylhydrazon-, Hydrazon- bzw. lminderivaten erfolgt dabei durch im Stand der Technik bekannte Verfahren.

Erfindungsgemäße Ligandenverbindungen, die eine Peptidbindung enthalten, können beispielsweise dadurch hergestellt werden, daß ein Peptid, das aus 2 bis etwa 30 Aminosäuren besteht, mit einer Trägermolekül-affinen Verbindung umgesetzt wird, so daß eine Trägermolekül-affine Verbindung direkt oder über ein Spacermolekül am N-terminalen Ende des Peptids eingeführt wird.

Die so erhaltenen Peptidderivate können mit Therapeutika und/oder Diagnostika bzw. deren Derivaten, die eine H₂N- oder HO-Gruppe besitzen, in Gegenwart eines Kondensationsmittels, wie beispielsweise N,N'-Dicyclohexylcarbodiimid (DCC), N-Cyclohexyl-N'-(2-morpholinoethyl)-carbodiimid-metho-p-toluolsulfonat (CMC), (Benzotriazol-1-yloxy)trispyrrolidinophosphonium-hexafluorophosphat (pyBOP) oder O-Benzotriazol-N,N,N',N'-tetramethyluronium-hexafluorophosphat, und gegebenenfalls unter Zusatz von einer Hydroxyverbindung, wie beispielsweise N-Hydroxysuccinimid, eines wasserlöslichen N-Hydroxysuccinimids, wie beispielsweise N-Hydroxysuccinimid-3-sulfonsäure-Natriumsalz oder 1-Hydroxybenzotriazol, und/oder in Gegenwart einer Base, wie beispielsweise N-Methylmorpholin oder Triethylamin, zu den entsprechenden erfindungsgemäßen Ligandenverbindungen umgesetzt werden:

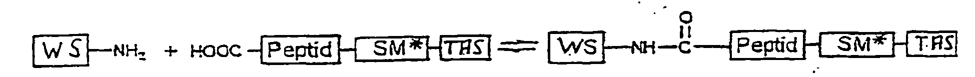

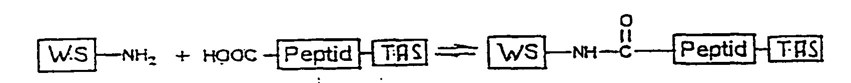

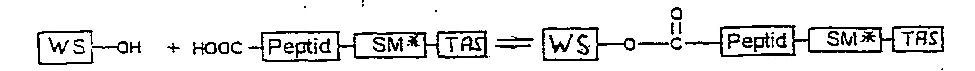

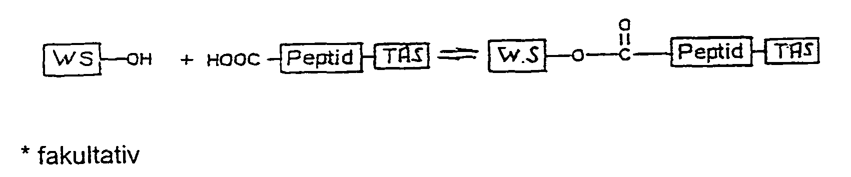

Die Substratspezifität von Target-Enzymen, wie etwa von MMP-2, MMP-3, MMP-9, Cathepsin B, D, L und H, ist bekannt (Netzel-Amett et al. (1993), *Biochemistry 32*, 6427-6432, Shuja, S., Sheahan, K., Mumame, M.J. *(1991), Int.J.Cancer 49,* 341-346, Lah, T.T., Kos, J. (1998), *Biol. Chem. 379*, 125-130).

Beispielsweise sind Octapeptide (P₄ - P'₄) für MMP-2 und MMP-9 (siehe Tabelle 1) identifiziert worden, welche die Spaltsequenz der Kollagenkette simulieren, und besonders effizient von MMP-2 und-9 gespalten werden:

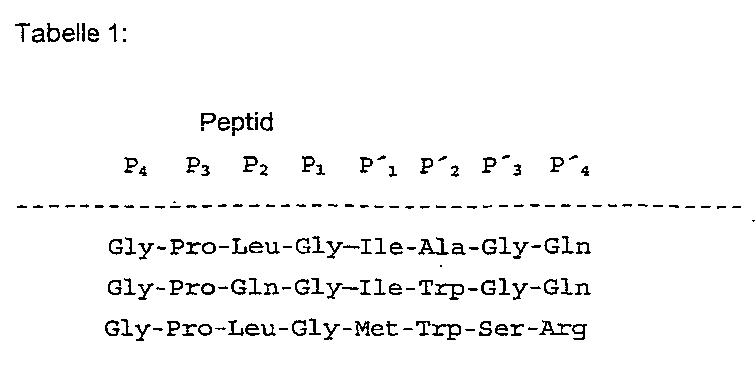

(Netzel-Arnett et al., *Biochemistry 32*, **1993**, 6427-6432)

Die Peptide werden ausschließlich an der P₁- P'₁-Bindung enzymatisch gespalten.

Beim Prostata-spezifischen Antigen (PSA) sind substratspezifische Hexapeptid-Sequenzen (P4-P2') bekannt, d.h. -His-Ser-Ser-Lys-Leu-Gln-, Asn-Ser-Ser-Tyr-Phe-Gln-oder-Ser-Ser-Tyr-Tyr-Ser-Gly- bekannt, die zwischen P1 und P1'durch PSA gespalten werden (Yang et al., J.Peptide Res. 54. 444-448, 1999; Denmeade et al., Cancer Res. 57, 4924-4930, 1997; Coombs et al., Chemistry & Biology 5, 475-488, 1998).

Des weiteren sind bei Cathepsin substratspezifsche Dipeptide mit der Sequenz -Arg-Arg-, -Phe-Lys-, Gly-Phe-Leu-Gly, Gly-Phe-Ala-Leu oder Ala-Leu-Ala-Leu bekannt (Werle, B., Ebert, E., Klein, W., Spiess, E. (1995), *Biol. Chem. Hoppe-Seyler 376*, 157-164; Ulricht, B., Spiess, E., Schwartz-Albiez, R., Ebert, W. (1995), *Biol. Chem. Hoppe-Seyler 376*, 404-414).

Die Peptidsequenz, welche die für das Target-Enzym relevante Peptidspalt- bzw. -sollbruchstelle enthält, kann auch so aufgebaut sein, daß die Peptidspaltstelle mehrfach wiederholt wird, wie beispielsweise durch:
-Gly-Pro-Leu-Gly―Ile-Ala-Gly-Gln-Gly-Pro-Leu-Gly―Ile-Ala-Gly-Gln
oder
-Phe-Lys-Phe-Lys-Phe-Lys-Phe-Lys-Phe-Lys-Phe-Lys-
oder es kann eine repetitive Peptidsequenz integriert werden, die den Abstand zwischen der thiolbindenden Gruppe und der relevanten Peptidspaltstelle vergrößert, wie beispielsweise durch:
-(Gly)ₙ-Phe-Lys-Phe-Lys-
mit vorzugsweise n = 2 bis 20, mehr bevorzugt n ≤ 12,
oder durch eine Oxaethylenglykol-Einheit als wasserlösliche Komponente
-(O-CH₂-CH₂)ₙ-Phe-Lys-Phe-Lys-
mit vorzugsweise n = 1 bis 8.

Ein wichtiges Merkmal dieser Ausführungsform der erfindungsgemäßen Ligandenverbindung ist die Tatsache, daß die für das jeweilige Target-Enzym relevante Peptidspaltstelle mindestens einmal in einem Oligopeptid, bestehend aus etwa 1 bis 30 Aminosäuren, vorkommt.

Die oben aufgeführten Oligopeptide sind repräsentative Beispiele für die enzymatisch spaltbare Bindung in den erfindungsgemäßen Ligandenverbindungen. Die oben genannten Proteasen sind Beispiele für krankeitsassoziierte Enyzme. Der therapeutische Ansatz kann selbstverständlich bei weiteren krankeitsassoziierte Proteasen angewandt werden.

Therapeutika- bzw. Diagnostikaderivate der erfindungsgemäßen Ligandenverbindungen, die ein Zytokin enthalten, können beispielsweise dadurch hergestellt werden, daß das Zytokin mit einer Spacermolekül-gekoppelten Trägermolekül-affinen Substanz, welche eine Carbonsäure oder eine aktivierte Carbonsäure aufweist, umgesetzt wird:

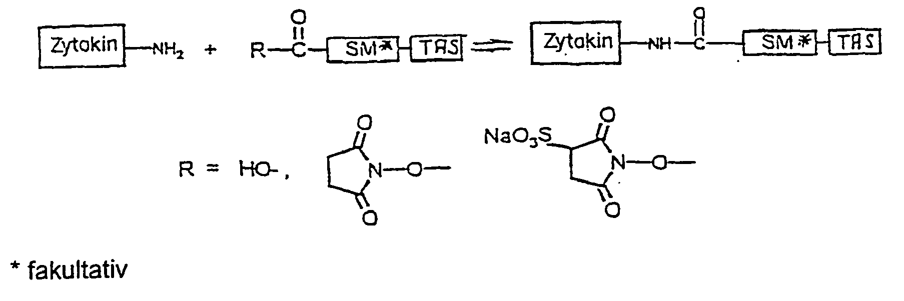

Weist das Spacermolekül eine N-Hydroxysuccinimidester-Gruppe (N-Hydroxysuccinimid oder Natriumsalz von N-Hydroxysuccinimid-3-sulfonsäure) auf, wird es direkt mit dem Zytokin umgesetzt. Die Umsetzung des Zytokins mit einer Spacermolekül-gekoppelten Trägermolekül-affinen Substanz, die eine Carbonsäure aufweist, erfolgt in der Gegenwart eines Kondensationsmittels, wie zum Beispiel N,N'-Dicyclohexylcarbodiimid (DCC) oder N-Cyclohexyl-N'-(2-morpholinoethyl)-carbodiimid-metho-p-toluolsulfonat (CMC), und gegebenenfalls unter Zusatz von N-Hydroxysuccinimid oder dem Natriumsalz der N-Hydroxy-succinimid-3-sulfonsäure, zu den entsprechenden Trägermolekül-affinen Zytokinderivaten. Die Aufreinigung der so derivatisierten Zytokine erfolgt beispielsweise mit Hilfe der Ausschlußchromatographie. Die oben beschriebenen Umsetzungen sind im Stand der Technik bekannt (siehe z.B. Bioconjugate Techniques, G.T. Hermanson; Academic Press, 1996).

Bevorzugte Trägermolekül-affine Substanzen in der erfindungsgemäßen Ligandenverbindung sind aus der Gruppe der Phthalocyanine, der Cumarine, der Flavonoide, der Tetracycline, der Naphthafene, der Aryl- und der Heteroarylcarbonsäuren, der Lipide und Fettsäuren, beispielsweise langkettige Fettsäuren wie C₁₆-C₂ₒ-Fettsäuren, cyclischen oder linearen Tetrapyrrole und deren metallorganischen Verbindungen, beispielsweise Porphyrine und Protoporphyrine (z.B. Bilirubin und dessen Derivate, Hämatin und dessen Derivate), der mit Halogenatomen (Cl, Br oder 1) 2-5-fach substituierten aromatischen Säurederivaten, wie lophenoxinsäure, der organischen Farbstoffe, beispielsweise Evans Blau und Bromkresol-Farbstoffe wie Bromkresol-grün und Bromkresolpurpur, und der Tryptophan- und Thyroxin-analogen Verbindungen sowie den Derivaten der vorstehend genannten Verbindungsklassen ausgewählt. Des weiteren können die als Trägermolekül-affine Substanzen verwendeten organischen Farbstoffe vor oder nach der Bindung an die therapeutisch und/oder diagnostisch wirksame Substanz bzw. an das Spacermolekül chemisch modifiziert bzw. derivatisiert werden, wobei jedoch das Bindungsverhalten im Vergleich zur nicht-modifizierten Verbindung mit dem Trägermolekül erhalten bleibt. Beispielsweise kann ein als Trägermolekül-affine Substanz verwendeter Farbstoff, z.B. ein Azofarbstoff, durch die vorstehend genannte chemischen Modifikation, beispielsweise durch Reduktion der Azogruppe oder durch Ersatz der Azogruppe durch eine C-C-Einfach- oder C-C-Doppelbindung, derart derivatisiert werden, daß er keine Farbe mehr aufweist.

Beispiele für Trägermolekül-affine Substanzen in der erfindungsgemäßen Ligandenverbindung sind aus folgenden Gruppen ausgewählt:

### Naphthalin- und Naphtylderivate: beispielsweise

1,4,5,8-NAPHTHALINTETRACARBONSÄURE, NAPHTHOL,
1,1'-(1,5-NAPHTHALINDIYL)BIS(3-CYCLOHEXYLHARNSTOFF),
4-ESSIGSÄUREAMIDO-5-HYDROXY-2,7-NAPHTHALIN-DISULFONSÄURE,
2-(2-HYDROXY-1-NAPHTHYLMETHYLENAMINO)BENZOESÄURE,
9-FLUORENYLIDENAMINO N-(1-NAPHTHYL)CARBAMAT,
2-(N-(1-NAPHTHYL)CARBAMOYL)CYCLOHEXANECARBONSÄURE,
1,1'-(1,5-NAPHTHALINDIYL)BIS(3-(3-PYRIDYLMETHYL)HARNSTOFF),
1,1'-(1,5-NAPHTHALINDIYL)BIS(3-BENZYLHARNSTOFF),
2,2'-OXYBIS(ESSIGSÄURE (2-HYDROXY-1-NAPHTHYLMETHYLEN)-HYDRA-ZID),
ADIPINSÄURE BIS((1-NAPHTHYLMETHYLEN)HYDRAZID),
3-(N-PHENYLCARBAMOYL)-2-NAPHTHYL N-(5-CHLORO-2-METHOXYPHENYL)CARBAMAT,
6-BENZOYL-2-NAPHTHYL PHOSPHAT, NATRIUM SALZ,
L-ARGININ 4-METHOXY-BETA-NAPHTHYLAMID HYDROCHLORID,
1-AMINO-8-NAPHTHOL-2,4-DISULFONSÄURE,
FMOC-D-2-NAPHTHYLALANIN, LYS-ALA 4-METHOXY-BETA-NAPHTHYLAMID DIHYDROCHLORID,
4-(1,8-DIHYDROXY-3,6-DISULFO-2-NAPHTHYLAZO)-SALICYLSÄURE,
1,1'-HEXAMETHYLENBIS(3-(1-NAPHTHYLMETHYL)HARNSTOFF,
CARBOBENZYLOXYTRYPTOPHAN BETA-NAPHTHYLAMID,
3-(N-(2-METHOXYPHENYL)CARBAMOYL)-2-NAPHTHYL N-(5-CHLORO-2-METHOXYPHENYL)CARBAMAT,
2-(1-NAPHTHYL)-2-PHENYLESSIGSÄURE, 8-[(2-CARBOXYETHYL)THIO]-1-NAPHTHOESÄURE,
3-[1-(2-CARBOXYETHYL)-2-OXO-1,2-DIHYDROACENAPHTHYLEN-1-YL]PROPANSÄURE, 3-[(1-NAPHTHYLAMINO)CARBONYL]BICYCLO[2.2.1]-HEPT-5-EN-2-CARBONSÄURE,
N",N"'-DI[1-(2-NAPHTHYL)ETHYLIDEN]KOHLENSÄURE DIHYDRAZID,
4-[[4-(2-NAPHTHYL)-1,3-THIAZOL-2-YL]AMINO]-4-OXOBUTANSÄURE,
FMOC-L-2-NAPHTHYLALANIN, 1,4,5,8-NAPHTHALINTETRACARBONSÄURE HYDRAT,
BIS(2-NAPHTHYL) N,N'-(4-METHYL-1,3-PHENYLEN)BISCARBAMAT,
8-HYDROXY-5-(1-NAPHTHYLAZO)-2-NAPHTHALINSULFONSÄURE, NATRIUM SALZ,
N'1,N'3-DI(1-NAPHTHYLMETHYLIDEN)-2-BUTYLPROPANDIOHYDRAZID,
(+/-)-1,1'-BINAPHTHYL-2,2'-DICARBONSÄURE,
(+/-)-2,2'-DIMETHOXY-1,1'-BINAPHTHYL-3,3'-DICARBOXAMID.
1-HYDROXY-4-(1-PHENYL-1H-TETRAZOL-5-YL-THIO)-2-NAPHTHOESÄURE, 3,3'-DIPHENYL-(1,1')BINAPHTHALENYL-2,2'-DICARBONSÄURE,
2,6-DIMETHOXY-4-OXO-3,5-DIOXA-PHOSPHA-CYCLOHEPTA(2,1-A,3,4-A')DINAPHTHALEN-4-OL,
4-((BR-4-ME-PHENYLIMINO)-ME)-3-HO-NAPHTHALIN-2-CARBONSÄURE (2-MEO-PH)-AMID,
8-([2-[(METHYLAMINO)CARBONYL]PHENYL]THIO)-1-NAPHTHOESÄURE,
N-GLUTARYL-L-PHENYLALANIN-BETA-NAPHTHYLAMID,
2-HYDROXY-3-NAPHTHOESÄURE-2-AMINOANTHRACHINONYLAMID PHOSPHAT,
2-HYDROXY-3-NAPHTHOESÄURE-2-AMINOAZOBENZANILID PHOSPHAT,
3-(5-(2-CARBOXY-ETHANSULFONYL)-NAPHTHALIN-1-SULFONYL)-PROPIONSÄURE,
4-((4-CL-PHENYLIMINO)ME)-3-HO-NAPHTHALIN-2-CARBONSÄURE (2-MEO-PH)-AMID,
4-HYDROXY-7-METHOXY-1-(3-METHOXY-PHENYL)-NAPHTHALIN-2-CARBONSÄURE,
3,6-DISULFO-NAPHTHALIN-1,8-DICARBONSÄURE, L-ALA-ALA-L-PHE-ALPHA-NAPHTHYLAMID,
CBZ-L-THR-L-VAL-BETA-NAPHTHYLAMID, CBZ-GLY-L-VAL-BETA-NAPHTHYLAMID,
CBZ-ILE-L-ALA-BETA-NAPHTHYLAMID, CBZ-L-ALA-THR-BETA-NAPHTHYLAMID,
DINATRIUM 4-(BENZOYLAMINO)-5-METHYL-2,7-NAPHTHALINDISULFONAT,
NAPHTHALIN-1,4,8-TRICARBONSÄURE,
2-[[2-(1-NAPHTHYL)ACETYL]AMINO]SUCCINSÄURE,
(10-HO-10-PH-4H-8-THIA-7,10A-DIAZA-PENTALENO(1,2-A)NAPHTHALEN-9-YL)-ESSIGSÄURE,
1-(NAPHTHALIN-2-CARBONYL)-3-NAPHTHALEN-2-YL-HARNSTOFF,
1-(3-METHOXY-PHENYL)-3-(NAPHTHALIN-1-CARBONYL)-THIOHARNSTOFF, (S)-N-(1-(1-NAPHTHYL) ETHYL)SUCCINAMIDSÄURE,
L-LYSYL-L-ALANIN-4-METHOXY-BETA-NAPHTHYLAMID HYDROBROMID,
5-((2-(T-BOC)-GAMMA-GLUTAMYLAMINOETHYL)AMINO)NAPHTHALIN-1-SULFONSÄURE,
8-([2-[(ACETYLOXY)METHYL]PHENYL]THIO)-1-NAPHTHOESÄURE,
2-[[(9H-FLUOREN-9-YLMETHOXY)CARBONYL]AMINO]-3-(2-NAPHTHYL)PROPANSÄURE,
1-NAPHTHYL-ACETYL SPERMIN TRIHYDROCHLORID,
4-[(2-ETHOXY-2-OXOETHYL)THIO]NAPHTHALIN-1,8-DICARBONSÄURE, FMOC-(R)-3-AMINO-4-(2-NAPHTHYL)-BUTYRIC ACID, 2,2'-BICHINONYL-DICARBONSÄURE;

### Benzophenonderivate: beispielsweise

BENZOPHENON-2,4'-DICARBONSÄURE,
3,3',4,4'-BENZOPHENONTETRACARBONSÄURE,
2',3,4-BENZOPHENONTRICARBONSÄURE,
2,2'-DIHYDROXY-4,4'-DIMETHOXY-5-SULFOBENZOPHENON,
4-[N-[2-(ESSIGSÄUREAMIDO)ETHYL]-N-METHYLAMINO]-2'-CARBOXY-2-HYDROXYBENZOPHENON;

### Phthal- und lsophthalimid-derivate und Phthal- und lsophthalsäurederivate: beispielsweise

5-SULFOISOPHTHALSÄURE MONONATRIUM SALZ,
2-PHENYL-3-PHTHALIMIDOQUINOLIN-4-CARBONSÄURE,
2-PHTHALIMIDOGLUTARSÄURE,
2,5-BIS(2-HYDROXYETHYLAMINO)TEREPHTHALSÄURE,
2- und/oder 4-SULFOTEREPHTHALSÄURE,
5-SULFO ISOPHTHALSÄURE,
2,5-BIS(N-(2,5-XYLYL)CARBAMOYL)TEREPHTHALSÄURE,
4-[(2-CARBOXYPHENYL)THIO]ISOPHTHALSÄURE,
4-(4-ACETYL-3-HYDROXY-2-PROPYLPHENOXY)ISOPHTHALSÄURE,
2,4,6-TRIMETHYLANILIN SALZ,
4-(N-(4-METHOXYPHENYL)CARBAMOYL)ISOPHTHALSÄURE,
PHTHALSÄURE MONO-((2-CHLORO-PHENYL)-PHENYL-METHYL) ESTER,
4-(4-(3,4-DtMETHYL-PHENYLSULFANYL)-BENZOLSULFONYL)-PHTHAL-SÄURE,
4-(4-(4-CARBOXY-PHENOXY)-BENZOYL)-PHTHALSÄURE,
4-(4-CHLORO-BENZOYL)-PHTHALSÄURE,
5-(5-(4-HYDROXY-PHENYL)-3-PHENYL-4,5-DIHYDRO-PYRAZOL-1-YL)-ISOPHTHALSÄURE,
5-(5-(4-DIMETHYLAMINO-PH)-3-PHENYL-4,5-DIHYDRO-PYRAZOL-1-YL)-ISOPHTHALSÄURE,
PHTHALSÄURE MONO-(BIPHENYL-4-YL-PHENYL-METHYL) ESTER,
PHTHALSÄURE MONO-(1-METHYL-2,2-DIPHENYL-ETHYL) ESTER,
4,5-BIS-(4-AMINO-PHENOXY)-PHTHALSÄURE,
4,5-DI(4-METHOXYPHENOXY)PHTHALSÄURE;

### Chinolin- und Isochinolinderivate: beispielsweise

N,N'-BIS(8-CHINOLYLOXYCARBONYL)-4-METHYL-1,3-PHENYLENDIAMIN, 3,3'-BIS(2-(2-CHINOLYL)VINYL)AZOBENZOL,
8-SULFO-2,4-CHINOLINDICARBONSÄURE,
2-[4-(ACETYLAMINO)PHENYL]-7-METHYLCHINOLIN-4-CARBONSÄURE,
3-(1,3-DIOXO-1H,3H-BENZO(DE)ISOCHINOLIN-2-YL)-BENZOESÄURE,
DINATRIUM 3-METHYL-4-(CHINOLIN-2-YLMETHYLEN)PENT-2-ENDIOAT,
2-(5-CARBOXY-PENTYL)-3-PH-1-PR-5,6,7,8-TETRAHYDRO-ISOCHINOLINIUM,
1-(1 H-INDOL-3-YL)-1H-ISOCHINOLIN-2-CARBONSÄURE PHENYLAMID,
1-ET-6-F-4-OXO-7-(4-(2-OXO-PR)PIPERAZIN-1-YL)-2H-CHINOLIN-3-CARBONSÄURE,
N-ETHYL-3-(PYRIDIN-2-YL)-4-(CHINOLIN-4-YL)PYRAZOL-1-CARBOXAMID,
2-[4-[(5-CHLORO-2-CHINOLYL)OXY]PHENOXY]PROPANSÄURE,
N2-[2-[(3-CYANO-4-METHYL-2-CHINOLYL)THIO]PHENYL]-2-FURAMlD,
3-(1-[1,1'-BIPHENYL]-4-YL-1H-1,2,3,4-TETRAAZOL-5-YL)CHINOLIN-4(1H)-ON, FMOC-BETA-(2-CHINOLYL)-ALA-OH,
N-BENZYL-2-[(4-METHYL-5-CHINOLIN-6-YL-4H-1,2,4-TRIAZOL-3-YL)THIO]-ESSIGSÄUREAMID,
FMOC-ALA(3'-CHINOYL)-OH,
FMOC-(S)-2-TETRAHYDROISOCHINOLIN ESSIGSÄURE;

### Anthrachinonderivate: beispielsweise

1,8-BIS(BENZAMIDO)ANTHRACHINON,
N,N'-1,5-ANTHRACHINONYLEN-DIANTHRANILSÄURE;

### Anthracenderivate: beispielsweise

2-(7-OXO-7H-BENZ(DE)ANTHRACEN-3-YLTHIO)ESSIGSÄURE,
9,10-DIHYDRO-9,10-DIOXO-2,3-ANTHRACENDICARBONSÄURE,
9-HYDROXYIMINO-9,10-DIHYDRO-ANTHRACEN-1-CARBONSÄURE;

### Phenanthren-und Phenanthrolinderivate: beispielsweise

1,10-PHENANTHROLIN-2,9-DICARBONSÄURE,
4-P-TOLYL-1,10-PHENANTHROLIN-2,9-DICARBONSÄURE,
1,2,3,4-TETRAHYDRO-PHENANTHREN-1,2-DICARBONSÄURE,

### Benzylidenderivate: beispielsweise

2,2'-OXYDIESSIGSÄURE BIS((4-ESSIGSAUREAMIDO-BENZYLIDEN)-HYDRAZID),
2-(FURFURYLUREIDO)ESSIGSÄURE N2-(4-HYDROXY-3-METHOXYBENZYLI-DEN)HYDRAZID,
2-(3-(3,4-DICHLOROPHENYL)UREIDO)ESSIGSÄURE (5-BROMO-2-HYDROXY-BENZYLIDEN)HYDRAZID,
2-(3-(2-ETHOXYPHENYL)UREIDO)ESSIGSÄURE (3,4-DIMETHOXYBENZYLI-DEN)HYDRAZID,
2-(3-(4-METHOXYPHENYL)UREIDO)ESSIGSÄURE (2,3,4-TRIMETHOXY-BENZYLIDEN)HYDRAZID,
4-(3-(2-FLUOROPHENYL)UREIDO)BENZOESÄURE (3,4-DICHLOROBEN-ZYLIDEN)HYDRAZID,
4-(3-(2-FLUOROPHENYL)UREIDO)BENZOESÄURE (4-METHYLBEN-ZYLIDEN)HYDRAZID,
4-(3-(2-FLUOROPHENYL)UREIDO)BENZOESÄURE (4-METHOXYBENZYLI-DEN)HYDRAZID,
4-(3-(2-FLUOROPHENYL)UREIDO)BENZOESÄURE (4-HYDROXY-3-METHOXY-BENZYLIDEN)HYDRAZID,
4-(3-(2-FLUOROPHENYL)UREIDO)BENZOESÄURE (4-ESSIGSÄUREAMIDO-BENZYLIDEN)HYDRAZID,
4-(3-(2-FLUOROPHENYL)UREIDO)BENZOESÄURE (3,4-DIMETHOXY-BENZYLIDEN)HYDRAZID,
2-(2-CHLOROBENZAMIDO)BENZOESÄURE (2-CHLORO-4-(DIME-THYLAMINO)-BENZYLIDEN)HYDRAZID,
2-(3-(3-CHLOROPHENYL)UREIDO)ESSIGSÄURE (4-BUTOXYBENZYLIDEN)-HYDRAZID,
2-(3-(6-METHYL-2-PYRIDYL)UREIDO)ESSIGSÄURE (3,4-DIMETHOXY-BEN-ZYLlDEN)HYDRAZID,
2-(3-(2-ETHOXYPHENYL)UREIDO)ESSIGSÄURE (2-HYDROXYBENZ-YLIDEN)HYDRAZID,
2-(3-(2-ETHOXYPHENYL)UREIDO)ESSIGSÄURE (4-ESSIGSÄUREAMIDO-BENZYLIDEN)HYDRAZID,
N'1-(4-CHLOROBENZYLIDEN)-3-[5-(2,3-DICHLOROPHENYL)-2H-1,2,3,4-TETRAAZOL-2-YL],
N'1-BENZYLIDEN-2-[3-[(4-METHYL-1,3-THIAZOL-2-YL)METHYL]-4-OXO-3,4-DIHYDROPHTHAL,
2,6-BIS(4-ESSIGSÄUREAMIDOBENZYLIDEN)-1-CYCLOHÖXANON,
N-BENZOYLANTHRANILSÄURE (4-HYDROXY-3-METHOXYBENZYLIDEN)-HYDRAZID,
4-(3-(3-CHLOROPHENYL)UREIDO)BENZOESÄURE (3-ETHOXY-4-HYDROXY-BENZYLIDEN)HYDRAZID,
4-(3-(3-CHLOROPHENYL)UREIDO)BENZOESÄURE (4-ETHOXY-3-METHOXY-BENZYLIDEN)HYDRAZID,
4-(3-(3-CHLOROPHENYL)URElDO)BENZOESÄURE(2-CHLORO-4-(DIMETHYL-AMINO)BENZYLIDEN)HYDRAZID,
4-(3-(3-CHLOROPHENYL)UREIDO)BENZOESÄURE (3,4-DICHLORO-BENZYLIDEN)HYDRAZID,
4-FLUOROBENZYL N-(4-METHOXYBENZYLIDEN)-[(ANILINOCARBONYL)-AMINO]-METHANHYDRAZONO,
2-((2-HO-BENZYUDEN)-AMINO)-5-(N'-(2-HO-BENZYLIDEN)-GUANIDINO)-PENTANSÄURE,
2,3-BIS-(3-METHOXY-BENZYLIDEN)-SUCCINSÄURE,
2,3-BIS-(3,4-DIMETHOXY-BENZYLIDEN)-SUCCINSÄURE,
2,3-BIS-(4-CHLORO-BENZYLIDEN)-SUCCINSÄURE,
2,3-DIBENZYLIDEN-SUCCINSÄURE,
BENZYLIDEN CBZ-NEURAMINSÄURE,
3-HYDROXY-2-((2-HYDROXY-BENZYLIDEN)-AMINO)-3-PHENYL-PROPIONSÄURE,
3-HO-3-(4-((2-HO-BENZYLIDEN)-AMINO)-PH)-2-PHENYLACETYLAMINO-PROPIONSÄURE;

### Di- und Biphenylderivate: beispielsweise

3-CHLORO-4-BIPHENYLYL N-(2,5-DIMETHOXYPHENYL)CARBAMAT,
2'-BENZOYL-2-BIPHENYLCARBONSÄURE,
2'-[(4-FLUOROANILINO)CARBONYL][1,1'-BIPHENYL]-2-CARBONSÄURE,
3-(2,4,6-TRIMETHYLBENZOYL)-2-BIPHENYLCARBONSÄURE,
2'-BENZYLCARBAMOYL-BIPHENYL-2-CARBONSAURE,
3-CHLORO-3'-METHYL-BIPHENYL-2,2'-DICARBONSÄURE,
2-HO-5-(4'-HO-BIPHENYL-4-YLAZO)-BENZOESÄURE,
2-[([1,1'-BIPHENYL]-4-YLAMINO)CARBONYL]BENZOESÄURE,
3-(1-[1,1'-BIPHENYL]-4-YL-1H-1,2,3,4-TETRAAZOL-5-YL)CHINOLIN-4(1H)-ON, 2-[[(3-[1,1'-BIPHENYL]-4-YL-3-OXOPROPYLIDEN)AMINO]OXY]-N-(5-IODO-2-PYRIDINYL)ESSIGSÄURE,
4,4'-DI-BOC-DIAMINOBIPHENYL-2,2'-DICARBONSÄURE,
2,3,2'-BIPHENYLTRICARBONSÄURE,
3-CHLORO-4-BIPHENYLYL N-(2,5-DIMETHOXYPHENYL)CARBAMAT,
4,4'-BIS(3-(3-PYRIDYL)UREIDO)DIPHENYLMETHAN,
4,4'-BIS(3-METHYL-3-PHENYLUREIDO)DIPHENYLMETHAN,
4,4'-METHYLENBIS(1,3-DIPHENYL-1-ETHYLHARNSTOFF),
2,2'-CARBOXYDIPHENYLSULPHON, 2,3-DIPHENYL-SUCCINSÄURE,
1,3-DIPHENYL-1-(2-MORPHOLINO-1-CYCLOPENTEN-1-YLCARBONYL)HARNSTOFF,
2-BENZOYLAMINO-3-(DIPHENYL-PHOSPHINOYL)-3-PHENYL-ACRYLSÄURE, MESO-2,3-DIPHENYLSUCCINSÄURE,
4,5-DIPHENYL-2,3-DIHYDRO-1H-PYRAZOLO[3,4-C]PYRIDAZIN-3-ON,
2-HYDROXY-2,2-DIPHENYL-ESSIGSÄURE (1-(4-BROMO-PHENYL)-ETHYLIDEN)-HYDRAZI D,
2,5-DIPHENYL-FURAN-3,4-DICARBONSÄURE,
1-(2,3-DIPHENYL-ACRYLOYL)3-(4-METHOXY-PHENYL)-THIOHARNSTOFF, N1-[2-(1,3-DIPHENYL-1 H-4-PYRAZOLYL)-1-(HYDRAZINOCARBONYL)VINYL]-2,4-DICHLOROBENZOESÄURE,
N1-[1-[(BENZYLAMINO)CARBONYL]-2-(1,3-DIPHENYL-'1 H-PYRAZOL-4-YL)VINYL]BENZAMID,
N2-ACETYL-06-(DIPHENYLCARBAMOYL)GUANIN;

### Tryptophan- und Indolderivate: beispielsweise

5-BENZYLOXYINDOL-3-ESSIGSÄURE,
6-BENZAMIDO-N-(2-(3-INDOLYL)ETHYL)HEXANAMID, N-(3-INDOLYLACETYL)-DL-ASPARAGINSÄURE,
INDOL-3-ACETYL-L-ASPARAGINSÄURE,
4-(3-INDOLYLMETHYLAMINO)BENZOESÄURE,
2-(2,2-BIS-(1H-INDOL-3-YL)-ETHYL)-PHENYLAMIN,
2-((5-BROMO-1,3-DIOXO-1,3-DIHYDRO-ISOINDOL-2-YLMETHYL)-AMINO)-BENZOESÄURE,
3-(2-(2-CARBOXY-BENZOYLAMINO)-ETHYL)-5-CHLORO-1H-INDOL-2-CARBONSÄURE,
3-PHENYL-PYRROLO(2,1,5-CD)INDOLIZIN-1,2-DICARBONSÄURE,
(R,S)-FMOC-1,3-DIHYDRO-2H-ISOINDOL CARBONSÄURE,
1-(1 H-INDOL-3-YL)-1H-ISOQUINOLIN-2-CARBONSÄURE PHENYLAMID,
N'-2-[[4-(DIMETHYLAMINO)PHENYL]METHYLEN]-3-PHENYL-1H-INDOL-2-CARBOHYDRAZID,
INDOL-3-ACETYL-DL-TRYPTOPHAN,
2-(1,3-DIOXO-1,3-DIHYDRO-2H-ISOINDOL-2-YL)-5-OXO-5-(4-TOLUIDINO)-PENTANSÄURE,
4-[(2-TERT-BUTYL-(-1H)-INDOL-5-YL)AMINO]-1-[(2-CHLOROPHENYL)-CARBONYL]PIPERIDIN,
2-[(2-CHLOROBENZOYL)AMINÖ]-N-[2-(1H-INDOL-3-YL)ETHYL]BENZAMID,
5-(4-CHLOROANILINO)-2-(1,3-DIOXO-1,3-DIHYDRO-2H-ISOINDOL-2-YL)-5-OXOPENTANSÄURE,
CARBOBENZYLOXY-L-METHIONYL-L-TRYPTOPHAN,
CARBOBENZYLOXY-L-PHENYLALANYL-L-TRYPTOPHANAMID,
N-ACETYL-5-BENZYLOXY-DL-TRYPTOPHAN,
CARBOBENZYLOXYTRYPTOPHAN BETA-NAPHTHYLAMID,
CARBOBENZYL-OXYNORVALYLTRYPTOPHANAMID,
L-NORLEUCYL-L-TRYPTOPHAN,
CARBOBENZYLOXY-L-TRYPTOPHYL-L-PHENYLALANINAMID,
CARBOBENZYLOXY-L-TRYPTOPHYL-L-LEUCINAMID,
CARBOBENZYLOXY-L-TRYPTOPHYLGLYCYLGLYCINE METHYL ESTER,
N-BETA-FMOC-L-BETA-HOMOTRYPTOPHAN

### Indanderivate: beispielsweise

4-SULFOBENZOESÄURE (5-INDANYLMETHYLEN)HYDRAZID,
PHENYLINDAN DICARBONSÄURE,
GLUTARSÄURE (5-INDANYLMETHYLEN)-HYDRAZID,
MALONSÄURE (5-INDANYLMETHYLEN)-HYDRAZID,
ADIPINSÄURE (5-INDANYLMETHYLEN)-HYDRAZID,
5-(5-INDANYLMETHYLENAMINO)-SALICYLSÄURE,
FMOC-2-AMINOINDAN-2-CARBONSÄURE;

### Hippursäurederivate: beispielsweise

N-ALPHA-HIPPURYL-L-HISTIDYL-L-LEUCIN,
P-HYDROXYHIPPURYL-HIS-LEU-OH,
HIPPURYL-HIS-LEU ACETAT SALZ,
HIPPURYL-ARG-GLY,
HIPPURYL-L-HISTIDYL-L-LEUCIN HYDRAT,
HIPPURYL-L-HISTIDYL-L-LEUCIN,
N-ALPHA-HIPPURYL-L-ARGININSÄURE HCL,
HIPPURYL-LYS-VAL-OH;

### Imidazol- und Benzimidazolderivate: beispielsweise

N-(2-(2-PYRIDYL)-5-BENZIMIDAZOLYL)MALEAMINSÄURE,
N-[2-(1H-BENZO[D]IMIDAZOL-2-YL)PHENYL]-N'-(4-METHOXYPHENYL)-THIOHARNSTOFF,
4-[4-(8-METHYLIMIDAZO[1,2-A]PYRIDIN-2-YL)ANILINO]-4-OXOBUTANSÄURE, 4-[6-[2-(4-CARBOXYPHENYL)-1 H-BENZO[D]IMIDAZOL-5-YL]-1 H-BENZO[D]IMIDAZOL-2-YL]BEN,
2-[3-(5-CARBOXY-1 H-BENZO[D]IMIDAZOL-2-YL)PHENYL]-1H-BENZO[D]IMIDAZOL-5-CARBOXYL,
5-(PIPERIDIN-1-CARBONYL)-1H-IMIDAZOL-4-CARBONSÄURE (2-BZ-4-BR-PH)-AMID,
2-PHENYLTHIOMETHYL-1-(2,4-DICHLOROANILINOCARBONYLMETHYL)-1H-BENZIMIDAZOL,
3-[6-ETHYL-7-HYDROXY-3-(1-METHYL-1H-BENZO[D]IMIDAZOL-2-YL)-4-OXO-4H-CHROMEN-2-YL,
1-(3,4-DIMETHYLPHENYLAMINOCARBONYLMETHYL)-2-(2-PHENYLVINYL)BENZIMIDAZOL,
2-(2-PHENYLVINYL)-1-(4-TRIFLUOROMETHOXYPHENYLAMINO-CARBONYL-METHYL)BENZIMIDAZOL,
FMOC-L-ALA-4-[5-(2-AMINO)IMIDAZOYL];

### Chinoxalinderivate: beispielsweise

2-(3-(BENZOLSULFONYL-CYANO-METHYL)-CHINOXALIN-2-YLOXY)-BENZOESÄURE;

### Pyridinderivate: beispielsweise

4,4'-BIS(3-(3-PYRIDYL)UREIDO)DIPHENYLMETHAN,
4,4'-METHYLENBIS(1-PHENYL-3-(3-PYRIDYLMETHYL)HARNSTOFF),
1,1'-HEXAMETHYLENBIS(3-(3-PYRIDYLMETHYL)HARNSTOFF),
4,4'-METHYLENBIS(N-(2-PYRIDYLOXYCARBONYL)ANILIN),
4,4'-METHYLENBIS(N-(3-PYRIDYLOXYCARBONYL)ANILIN),
4,4'-METHYLENBIS(1-PHENYL-3-(4-PYRIDYLMETHYL)HARNSTOFF),
4-(3-(2-FLUOROPHENYL)UREIDO)BENZOESÄURE (2-PYRIDYL-METHYLEN)-HYDRAZID,
2-PHENYL-2-[2-(4-PYRI DYLCARBONYL)HYDRAZONO]ESSIGSÄURE,
N1-[2-(2,4-DICHLOROPHENOXY)PHENYL]-N2-(3-PYRIDYLMETHYL)-ETHANDIAMID,
N'-[(ANILINOCARBONYL)OXY]-3-[(PYRIDIN-2-YLSULFONYL)METHYL]-BENZOLCARBOXIMIDAMID,
N'-([[(2-CHLOROPYRIDIN-3-YL)AMINO]CARBONYL]OXY)-3-[(PYRIDIN-2-YLSULFONYL)METHYL],
N1-(4-ISOPROPYLPHENYL)-2-[[4-(3-PYRIDYL)PYRIMI DI N-2-YL]THIO]ESSIG-SÄUREAMID,
2-[([2-[(4-METHYLPHENYL)THIO]-3-PYRIDYL]CARBONYL)AMINO]-ESSIGSÄURE,
2-([[2-(4-CHLOROPHENOXY)-3-PYRIDYL]CARBONYL]AMINO)ESSIGSÄURE,
2-[([2-[(4-CHLOROPHENYL)THIO]-3-PYRIDYL]CARBONYL)AMINO]-ESSIGSÄURE,
N-(3-CHLOROPHENYL)-N'-[6-[(5-CHLORO-3-PYRIDYL)OXY]-3-PYRIDYL]-HARNSTOFF,
N-(2,6-DICHLOROBENZOYL)-N'-[6-(3-PYRIDYLOXY)-3-PYRIDYL]HARNSTOFF,
ETHYL 2-[(5-CHLORO-3-PYRIDYL)OXY]-5-[[(3,4-DICHLOROANILINO)CARBONYL]AMINO]BENZOAT,
N1-(3-PYRIDYLMETHYL)-2-[[(2-CHLOROANILINO)CARBOTHIOYL]-AMINO]BENZAMID,
N-[[6-(4-CHLOROPHENOXY)-3-PYRIDYL]CARBONYL]-N'-(4-CHLOROPHENYL)-HARNSTOFF,
N-(2-CHLOROPHENYL)-N'-[5-(2-PHENYLETH-1-YNYL)-3-PYRIDYL]-HARNSTOFF,
4-BENZYLAMINO-5-CYANO-6-(4-METHOXYPHENYL)-2-(4-PYRIDYL)-PYRIMIDIN,
N-(2-CHLOROPHENYL)-N'-[[5-(2-PHENYLETH-1-YNYL)-3-PYRIDYL]-CARBONYL]THIOHARNSTOFF,
N-(4-METHOXYPHENYL)-N'-[[5-(2-PHENYLETH-1-YNYL)-3-PYRIDYL]-CARBONYL]THIOHARNSTOFF,
FMOC-L-2-PYRIDYLALA,
N1-PHENYL-3-[2-(6-FLUORO-2-PYRIDYL)HYDRAZONO]-2-HYDROXY-IMINOBUTANAMID,
2-[([6-[(4-METHYLPHENYL)THIO]-3-PYRIDYL]AMINO)CARBONYL]CYCLOPROPAN-1-CARBONSÄURE,
N'1-[3-(1H-PYRROL-1-YL)-2-PYRIDYL]-3,4-DICHLOROBENZOL-1-SULFONOHYDRAZID,
4-(METHYLNITROSAMINO)-1-(3-PYRIDYL)-1-BUTANYL BETA-D-GLU-CURO-NID,
4-(2,4-DICHLOROPHENYL)-5-(2-PHENYL-1-DIAZENYL)-2-(3-PYRIDYL)-PYRIMIDIN;

### Cumarinderivate: beispielsweise

N-EPSILON-CBZ-L-LYSIN 7-AMIDO-4-METHYLCUMARIN HYDROCHLORID,
7-METHOXYCUMARIN-4-ACETYL-PRO-LEU, CUMARIN 6, CUMARIN 7, CUMARIN 138, CUMARIN 152, CUMARIN 314, CUMARIN 334, CUMARIN 337, CUMARIN 343;

### Flavon- und Flavonoidderivate: beispielsweise

3'-BENZYLOXY-5,7-DIHYDROXY-3,4'-DIMETHOXYFLAVON,
FLAVON-3-DIPHOSPHAT,
3'-BENZYLOXY-5,7-DIHYDROXY-3,4'-DIMETHOXYFLAVON;

### Pyrimidinderivate: beispielsweise

3-[(4-CHLORO-6-METHYLPYRIMIDIN-2-YL)AMINO]-2-[3-[(PHENYLTHIO)-METHYL]BENZOYL]ACRYL,
N-(4-CHLOROPHENYL)-N'-[3-[(2,5,6-TRICHLOROPYRIMIDIN-4-YL)AMINO]-PHENYL]HARNSTOFF,
2-BENZOYLAMINO-3-(2'-4-METHYLPYRIMIDYL)-AMINOPROPENOYL HYDRAZID-4-CHLOROBENZALDEHYD,
2-BENZOYLAMINO-3-(2'-(4'-METHYLPYRIMIDYL)-AMINOPROPENOYL HYDRAZID-4-BENZALDEHYDEHYD,
2-BENZOYLAMINO-3-(2'-(4'-METHYLPYRIMIDYL)-AMINOPROPENOYL HYDRAZID-4-METHYLBENZALDEHYD,
2-BENZOYLAMINO-3-(2'-(4'-METHYLPYRIMIDYL)-AMINOPROPENOYL HYDRAZID-4-METHOXYBENZALDEHYD,
2-BENZOYLAMINO-3-(2'-(4'-METHYLPYRIMIDYL)-AMINOPROPENOYL HYDRAZON-4-FLUOROBENZA,
3-((4-METHYLPYRIMIDYL)-2-AMINO)-2-BENZOYLAMINO ACRYLOYL HYDRA-ZIDDIMETHYLAMINO,
4-[[4-[(4,6-DIMETHYLPYRIMIDIN-2-YL)OXY]PHENYL]AMINO]-4-OXO-(2Z)-BUTENSÄURE,
4-[[4-[(4,6-DIMETHYLPYRIMIDIN-2-YL)THIO]PHENYL]AMINO]-4-OXO-(2Z)-BUTENSÄURE,
(2-AMINO-5-(3-AMINO-4-CHLORO-PHENYL)-PYRIMIDIN-4-YLSULFANYL)-ESSIGSÄURE,
2-HO-BENZOESÄURE (2-(5-ET-HO-ME-PYRIMIDIN-YLSULFANYL)-PH-ETHYLIDEN)-HYDRAZID,
(4-(3-(4-MEO-PHENYL)-THIOUREIDO)-6-METHYL-PYRIMIDIN-2-YLSULFANYL)-ESSIGSÄURE,
4-(2,4-DICHLOROPHENYL)-2-PHENYL-5-(2-PHENYL-1-DIAZENYL)PYRIMIDIN, N-(4-CHLOROPHENYL)-N'-[(1,3-DIBENZYLHEXAHYDROPYRIMIDIN-5-YL)METHYL]HARNSTOFF,
N-(2-CHLOROBENZOYL)-N'-[(1,3-DIBENZYLHEXAHYDROPYRIMIDIN-5-YL)METHYL]HARNSTOFF,
FMOC-2-AMINO-4-[(2-AMINO)PYRIMIDINYL]BUTANSÄURE,
4-OXO-4-([2-[(5-PHENYLTHIENO[2,3-D]PYRIMIDIN-4-YL)AMINO]-ETHYL]AMINO)BUTANSÄURE;

### Piperidinderivate: beispielsweise

N,N'-(METHYLENDI-4,1-PHENYLEN)BIS(3-METHYL-1-PIPERIDIN-CARBOX-AMID),
N,N'-(METHYLENDI-4,1-PHENYLEN)BIS(4-METHYL-1-PIPERIDINCARBOX-AMID),
N-[[1-(3-CHLORO-2-CYANOPHENYL)-4-PIPERIDYL]CARBONYL]-N'-(2,6-DICHLOROPHENYL)HARNSTOFF,
N4-(1-BENZYL-4-PIPERIDYL)-3-(2,6-DICHLOROPHENYL)-5-METHYLISOXAZOL-4-CARBOXAMID,
1,4-BIS(2-CARBOXYBENZOYL)PIPERAZIN,
5-(PIPERIDIN-1-CARBONYL)-1 H-IMIDAZOL-4-CARBONSÄURE (2-BZ-4-BR-PH)-AMID,
FMOC-4-CARBOXYMETHYL-PIPERAZIN,
4-FMOC-PIPERAZIN-1- YLESSIGSÄURE,
2-[[2-(4-METHYLPIPERAZINO)PHENYL]METHYLEN]MALONSÄURE,
1-BOC-PIPERIDIN-4-FMOC-AMINO-4-CARBONSÄURE,
5-[(4-CHLOROBENZOYL)AMINO]-2-(3,5-DIMETHYLPIPERIDINO)BENZOESÄURE,
4-CHLORO-N-[4-[4-(2-METHOXYPHENYL)PIPERAZINO]BUTYL]BENZAMID,
FMOC-L-ALA[3-(1-N-PIPERAZINYL(4-N-BOC))];

### Sarcosinderivate: beispielsweise

CARBOBENZYLOXYSARCOSYL-L-ALANIN,
CARBOBENZYLOXYGLYCYLGLYCYLSARCOSIN;

### Oxazol-, Oxadiazol- und lsooxazolderivate: beispielsweise

N1-(3-CHLOROPHENYL)-5-(3-PHENYLISOXAZOL-5-YL)-1H-PYRAZOL-1-CARBOXAMID,
N1-[2-[3-(4-CHLOROPHENYL)-1,2,4-OXADIAZOL-5-YL]PHENYL]-3,4-DICHLOROBENZAMID,
2-([2-[4-(1,3-OXAZOL-5-YL)ANILINO]-2-OXOETHYL]THIO)ESSIGSÄURE,
N-(5-METHYLISOXAZOL-3-YL)-N'-[(5-PHENYL-1,3,4-OXADIAZOL-2-YL)CARBONYL]HARNSTOFF,
N'-([[(2-FLUOROBENZOYL)AMINO]CARBONYL]OXY)-N-(5-METHYLISOXAZOL-3-YL)IMINOFORMAMID,
N-[3-(2-CHLOROPHENYL)-5-METHYLISOXAZOL-4-YL]-N'-(3,4-DIMETHOXY-PHENETHYL)HARNSTOFF,
N4-[2-CHLORO-5-(1-HYDROXYIMINOETHYL)PHENYL]-3-(2-CHLOROPHENYL)-5-METHYLISOXAZOL,
2,6-DICHLORO-N'-[([[3-(2,6-DICHLOROPHENYL)-5-METHYLISOXAZOL-4-YL]AMINO]CARBONYL);

### Pyrazol-, Tri- und Tetraazolderivate: beispielsweise

1-(2,5-DICHLORO-4-SULFOPHENYL)-5-PYRAZOLON-3-CARBONSÄURE, CARBOXYPYRAZOLON-2,5-DISULFONSÄURE,
N-[4-[(4-CHLOROPHENYL)SULFONYL]-3-METHYL-2-THIENYL]-N'-(3-CYCLOPROPYL-1 H-PYRAZOL,
N 1-(1,3,5-TRIMETHYL-1H-PYRAZOL-4-YL)-2-[[(4-CHLOROANILINO)CARBONYL]AMINO]BENZAMID;
N-BENZOYL-N'-[3-CYCLOPROPYL-4-[2-(3,4-DICHLOROPHENYL)DIAZ-1-ENYL]-1H-PYRAZOL-5-Y,
1,5,1',5'-TETRAPHENYL-1 H,1'H-(3,3')BIPYRAZOLYL,
2-[[1-(4-METHOXYPHENYL)-3-METHYL-1H-PYRAZOL-5-YL]AMINO]BENZOESÄURE,
N1-[2-(1,3-DIPHENYL-1H-4-PYRAZOLYL)-1-(HYDRAZINOCARBONYL)VINYL]-2,4-DICHLOROBENZAMID,
N1-[1-[(BENZYLAMINO)CARBONYL]-2-(1,3-DIPHENYL-1H-PYRAZOL-4-YL)VINYL]BENZAMID, 1-(3-CHLOROPHENYL)-5-(4-CHLOROPHENYL)-1 H-PYRAZOL-3-CARBONSÄURE,
5-(4-CHLOROPHENYL)-1-(2,5-DICHLOROPHENYL)-1H-PYRAZOL-3-CARBONSÄURE,
5-(2-CHLOROPHENYL)-1-(2,4-DICHLOROPHENYL)-1H-PYRAZOL-3-CARBONSÄURE,
5-(2-CHLOROPHENYL)-1-(3-CHLOROPHENYL)-1 H-PYRAZOL-3-CARBONSÄURE,
5-(2-CHLOROPHENYL)-1-(4-CHLOROPHENYL)-1 H-PYRAZOL-3-CARBONSÄURE,
5-(2-CHLOROPHENYL)-1-(3,4-DICHLOROPHENYL)-1 H-PYRAZOL-3-CARBONSÄURE,
N,5-BIS(4-CHLOROPHENYL)-1-(2,5-DICHLOROPHENYL)-1H-PYRAZOL-3-CARBOXAMID,
1-(4-CHLOROPHENYL)-5-(3,4-DICHLOROPHENYL)-1 H-PYRAZOL-3-CARBONSÄURE,
1-(4-CHLOROPHENYL)-5-PHENYL-1 H-PYRAZOL-3-CARBONSÄURE,
1-(2,4-DICHLOROPHENYL)-5-PHENYL-1 H-PYRAZOL-3-CARBONSÄURE,
1-(4-METHYLPHENYL)-5-PHENYL-1 H-PYRAZOL-3-CARBONSÄURE,
1-(4-FLUOROPHENYL)-5-PHENYL-1 H-PYRAZOL-3-CARBONSÄURE,
5-(2-CHLOROPHENYL)-N-(3,4-DIMETHYLPHENYL)-1 -PHENYL-1 H-PYRAZOL-3-CARBOXAMID,
1-(2-CHLOROPHENYL)-5-PHENYL-1-H-PYRAZOL-3-CARBONSÄURE,
1-[2-(2,4-DICHLOROPHENOXY)PHENYL]-5-PHENYL-1H-1,2,3-TRIAZOL-4-CARBONSÄURE,
1-BENZYL-3-[2-[(1-METHYL-5-CARBOXYTHIOMETHOXY)-1,3,4-TRIAZOLYL]]-2-PYRIDON,
O-(2,4-DICHLOROBENZYL)-N-[1-PHENYL-1,2,4-TRIAZOL-3-OXYACETYL]FORMADOXIM,
FMOC-L-TRANSPRO(4-TETRAZOYL);

### Thiazol- und Benzothiazolderivate: beispielsweise

1,1'-(4-METHYL-1,3-PHENYLEN)BIS(3-(2-BENZOTHIAZOLYL)HARNSTOFF), 4,4'-METHYLENBIS(1-PHENYL-3-(2-THIAZOLYL)HARNSTOFF),
4-CHLORO-N'-[([[2-(2,3-DICHLOROPHENYL)-1,3-THIAZOL-4-YL]AMINO]CARBONYL)OXY]BENZOL,
N-PHENYL-N-(4-CHLOROPHENYL)URIDO-3-(2-ETHOXYCARBONYLPHENYL)-1-AMINO THIAZOL,
2-(2,5-DIHYDROXYPHENYLTHIO)BENZOTHIAZOL-5-SULFONSÄURE,
3-CARBOXYMETHYL-2-CARBOXYMETHYLSULFANYL-BENZOTHIAZOL-3-IUM, CHLORID,
3-[6-(2-CARBOXYETHYL)[1,3]THIAZOLO[5',4':4,5]BENZO[D][1,3]THIAZOL-2-YL]PROPANSÄURE,
2-(2-(4-CHLOROPHENYL)-4-PHENYLTHIAZOL-5-YL)ESSIGSÄURE,
N-(2-BENZYLSULFANYL-BENZOTHIAZOL-6-YL)-SUCCINAMIDSAURE, 2-(2-[[2-(1,3-BENZOTHIAZOL-2-YLSULFANYL)ACETYL]AMINO]-1,3-THIAZOL-4-YL)ESSIGSÄURE;

### Triazinderivate: beispielsweise

N1-[4-(BENZOYLAMINO)-1-PHENYL-6-(PHENYLIMINO)-1,6-DIHYDRO-1,3,5-TRIAZIN-2-YL],
2-(3,5-DIOXO-2,3,4,5-TETRAHYDRO-(1,2,4)TRIAZIN-6-YLAMINO)-PENTANDISÄURE,
06-(4-CHLOROBENZOYL)-3,5-DIOXO-2-PHENYL-4-PROPYL-2,3,4,5-TETRAHYDRO-1,2,4-TRIAZIN,
3-[3,5-DI(TERT-BUTYL)PHENYL]-N-(4-METHOXY-6-METHYL-1,3,5-TRIAZIN-2-YL)-2,2-DIOXO;

### Morphotinderivate: beispielsweise

4,4'-METHYLENBIS(N-(2,6-DIMETHYLMORPHOLINOCARBOXAMIDO)ANILIN),
1,1'-HEXAMETHYLENBIS(3-(2-MORPHOLINOETHYL)HARNSTOFF),
1,3-DIPHENYL-1-(2-MORPHOLlNO-1-CYCLOPENTEN-1-YLCARBONYL)HARNSTOFF,
N-(2-(4-DIMETHYLAMINO-PHENYL)-1-(MORPHOLIN-4-CARBONYL)-VINYL)-BENZAMID,
METHYL 5-[[(3,5-DICHLOROANILINO)CARBONYL]AMINO]-2-MORPHOLINO-BENZOESÄURE,
FMOC-2-CARBOXYMORPHOLIN,
N-(2-MORPHOLINOETHYL)-4-OXO-4-[(2,3,6,7-TETRAHYDRO-(1H,5H)-BENZO[IJ]CHINOLIZIN;

### Chromenderivate: beispielsweise

2-([2-OXO-2-[(4-OXO-2-PHENYL-4H-CHROMEN-3-YL)AMINO]ETHYL]THIO)ESSIGSÄURE,
N1-(4-OXO-2-PHENYL-4H-CHROMEN-3-YL)-2-(ETHYLSULFONYL)ESSIGSÄUREAMID,
2-OXO-2-[(4-OXO-2-PHENYL-4H-CHROMEN-3-YL)AMINO]ESSIGSÄURE;

### Pyrrol- und Di-, Tri-, und Tetrapyrrolderivate und deren metallorganischen Verbindungen, d.h. Porphyrine und Protoporphyrine: beispielsweise

3-[1-CYANO-3-(2-PHENYLHYDRAZONO)PROP-1-ENYL]-1-PHENYL-5-(1H-PYRROL-1-YL),
N'-([[(2,6-DICHLOROBENZOYL)AMINO]CARBONYL]OXY)-N-[2,5-DICHLORO-4-(1H-PYRROL-1-YL,
N-[4-(2,4-DICHLOROBENZOYL-2-PYRROLYL]-N'-PHENYL HARNSTOFF,
O-(2,4-DICHLOROBENZYL)-N-[4-(2,4-DICHLOROBENZOYL)PYRROL-2-CARBONYL]FORMAMIDOXIM,
5-(4-METHYLPHENYL)-2,3-DIHYDRO-1H-PYRROLIZIN-6,7-DICARBONSÄURE, 5-PHENYL-2,3-DIHYDRO-1 H-PYRROLIZIN-6,7-DICARBONSÄURE,
5-(3-CHLOROPHENYL)-2,3-DIHYDRO-1H-PYRROLIZIN-6,7-DICARBONSÄURE, 5-(4-CHLOROPHENYL)-2,3-DIHYDRO-1H-PYRROLIZIN-6,7-DICARBONSÄURE, 5-(4-FLUOROPHENYL)-2,3-DIHYDRO-1H-PYRROLIZIN-6,7-DICARBONSÄURE, 5-(4-METHOXYPHENYL)-2,3-DIHYDRO-1 H-PYRROLIZIN-6,7-DICARBONSÄURE,
3-PHENYL-PYRROLO(2,1,5-CD)INDOLIZIN-1,2-DICARBONSÄURE,
1-(2-BENZO[1,3]DIOXOL-5-YL-ET)-2-(2-CARBOXY-ET)-OXO-PYRROLIDIN-CARBONSÄURE,
FMOC-4-AMINO-1-BOC-PYRROLIDIN-2-CARBONSÄURE,
1-[(9H-FLUOREN-9-YLMETHOXY)CARBONYL]PYRROLIDIN-2-CARBONSÄURE,
1-(TERT-BUTOXYCARBONYLAMINOETHYL)-2-METHYL-5-PHENYLPYRROLE-3-CARBONSÄURE,
2-N'-FMOC-AMINOMETHYLPYRROLIDIN-N-ESSIGSÄURE,
3-N'-FMOCAMINOPYRROLIDIN-N-ESSIGSÄURE,
(2S, 4RS)-4-FMOC-AMINO-1-BOC-PYRROLIDIN-2-CARBON SÄURE, BILIRUBIN, HÄMATIN;

### Tri-, Tetra-, Pentasubstituerte Ethan- bzw. Propansäurederivate: beispielsweise

N-(2-HYDROXYETHYL)ETHYLENDIAMINTRIESSIGSÄURE,
N-(2-HYDROXYETHYL)ETHYLENDIAMIN-N,N',N'-TRIESSIGSÄURE,
ETHYLENBIS(OXYETHYLENNITRILO)]TETRAESSIGSÄURE,
1,6-DIAMINOHEXANE-N,N,N',N'-TETRAESSIGSÄURE,
N-(2-HYDROXYETHYL)ETHYLENDIAMINTRIESSIGSÄURE,
ETHYLENDIAMINTETRAESSIGSÄURE,
2-OXO-1,1,3,3-CYCLOHEXANETETRAPROPIONSÄURE,
N,N,N',N'-1,4-PHENYLENDIAMINTETRAESSIGSÄURE,
ETHYLEN GLYCOL BIS(BETA-AMINOETHYLETHER)-N,N,N',N'-TETRAESSIGSÄURE,
ETHYLENGLYCOL-BIS(BETA-AMINOETHYLETHER)-N,N,N',N'-TETRAESSIGSÄURE,
DIETHYLENTRIAM1NPENTAESSIGSÄURE;

### Stilbenderivate: beispielsweise

2,3,5,4'-TETRAHYDROXYSTILBEN-2-O-BETA-D-GLUCOSID,
4,4' DIHYDRAZINO STILBEN 2-2' DISULFONSÄURE,
4-ACETAMIDO-4'-ISOTHIOCYANATOSTILBEN-2,2'-DISULFONSÄURE;

Mit Halogenatomen (C1, Br oder I) 2-5-fach substituierten aromatischen Verbindungen: beispielsweise 1-(4-BENZOYLPHENYL)-3-(2,4-DICHLOROPHENYL)HARNSTOFF, N,N'-(4-(ALPHA-CYANO-2,4-DICHLOROSTYRYL)-1,2-PHENYLEN)-BISESSIGSÄUREAMID, 1-(2-BENZOYL-4-CHLOROPHENYL)-3-(2,5-DICHLOROPHENYL)HARNSTOFF, 1-(4-BENZOYLPHENYL)-3-(2,5-DICHLOROPHENYL)HARNSTOFF, 3',4'-DICHLOROPHTHALANILINSÄURE, 1-(2-BENZOYL-4-CHLOROPHENYL)-3-(2,6-DICHLOROPHENYL)HARNSTOFF, 1-(2-BENZOYL)-4-CHLOROPHENYL)-3-(2,3-DICHLOROPHENYL)HARNSTOFF, 1-(2-CARBOXYPHENYL)-3-(2,3-DICHLOROPHENYL)HARNSTOFF, 1-(2-CARBOXYPHENYL)-3-(3,4-DICHLOROPHENYL)HARNSTOFF, 2-[(2-CARBOXY-4,6-DICHLOROPHENYL)THIO]-3,5-DICHLOROBENZOESÄURE, 4-[2-(2,4-DICHLOROPHENOXY)ANILINO]-4-OXOBUT-2-ENOIC ACID, 4-[2-(2,4-DICHLOROPHENOXY)ANILINO]-4-OXOBUTANSÄURE, N-BENZOYL-N'-[2-(2,4-DICHLOROPHENOXY)PHENYL]THIOHARNSTOFF, 3-[(3,5-DICHLOROANILINO)CARBONYL]PYRAZIN-2-CARBONSÄURE, N1-(4-FLUOROPHENYL)-2-[[(3,4-DICHLOROANILINO)CARBOTHIOYL]AMINO]BENZAMID, N-[2-(2,4-DICHLOROPHENOXY)PHENYL]-N'-(2,6-DIFLUOROBENZOYL)HARNSTOFF, N-(2-CHLORO-6-FLUOROBENZOYL)-N'-[2-(2,4-DICHLOROPHENOXY)PHENYL]HARNSTOFF, N1-[2-[5-(3,4-DICHLOROPHENYL)-1,3,4-OXADIAZOL-2-YL]PHENYL]-3-METHYLBENZAMID, ETHYL 2-[(5-CHLORO-3-PYRIDYL)OXY]-5-[[(3,4-DICHLOROANILINO)CARBONYL]AMINO]BENZOESÄURE, N-[2-(2,4-DICHLOROPHENOXY)BENZOYL]-N'-(2,4-DIFLUOROPHENYL)HARNSTOFF, N-[2-(2,4-DICHLOROPHENOXY)BENZOYL]-N'-(3,5-DICHLOROPHENYL)HARNSTOFF, 3-[[(2,5-DICHLOROANILINO)CARBOTHIOYL]AMINO]PROPANSÄURE, N1-(4-[[(3,4-DICHLOROANILINO)CARBOTHIOYL]AMINO]PHENYL)-2-HYDROXYBENZAMID, N1-(4-[[(2,3-DICHLOROANILINO)CARBOTHIOYL]AMINO]PHENYL)-2-HYDROXYBENZAMID, N1-(3-[[(3,4-DICHLOROANILINO)CARBOTHIOYL]AMINO]PHENYL)-2-HYDROXYBENZAMID, 1-(1-(3,4-DICHLOROBENZYL))-3-(2-METHOXYCARBONYLPHENYL)UREIDO-2-PYRIDON, 1-(1-(3,4-DICHLOROBENZYL))-3-(3-ISOPROPOXYPHENYL)UREIDO-2-PYRIDON, BIS-(4-METHYLPHENYLAMINO)MALONAMID-2-(2,4-DICHLOROBENZYL OXIM)-METHYLEN, 4-(3-(3-CHLOROPHENYL)UREIDO)BENZOESÄURE (3,4-DICHLOROBENZYLIDEN)HYDRAZID, 3-(N-(3,4-DICHLOROPHENYL)CARBAMOYL)-5-NORBORNEN-2-CARBONSÄURE, 2-CARBOXYMETHYLTHIO-2',4'-DICHLORO-2-PHENYLESSIGSÄUREANILID, O-(2,4-DICHLOROBENZYL)-N-[1-PHENYL-1,2,4-TRIAZOL-3-OXYACETYL]FORMADOXIM, 3,3-DICHLORO-2-(4-CHLORO-BENZOYLAMINO)-ACRYLLSÄURE, 2-[[2-(3,5-DICHLOROANILINO)-2-OXOETHYL]THIO]NICOTINSÄURE, 1-[[(2,3-DICHLOROANILlNO)CARBOTHIOYL]AMINO]ETHYLPHOSPHONSÄURE, 2-[[2-(2,4-DICHLORO-5-ISOPROPOXYANILINO)-2-OXOETHYL]SULFANYL]ESS1GSÄURE, 2-([2-[5-(BENZYLOXY)-2,4-DICHLOROANILINO]-2-OXOETHYL]SULFANYL)ESSIGSÄURE, 2-([2-[2,4-DICHLORO-5-(2-PROPYNYLOXY)ANILINO]-2-OXOETHYL]SULFANYL)ESSIGSÄURE, 2,4-DICHLORO-N-[3-[(2,4-DICHLOROBENZOYL)AMINO]-3-OXOPROPANOYL]BENZOLCARBOXAMID, FMOC-(R)-3-AMINO-4-(3,4-DICHLORO-PHENYL)-BUTTERSÄURE, FMOC-2,4-DICHLORO-L-PHENYLALANIN, 1-(2-BENZOYL-4-CHLOROPHENYL)-3-(2,4,5-TRICHLOROPHENYL)HARNSTOFF, 3-(3-(2,4,5-TRlCHLOROPHENYL)UREIDO)BENZOESÄURE, N1-(4-FLUOROPHENYL)-2-(BENZOYLAMINO)-3,5-DIBROMOBENZAMID, 2,3,5-TRIIODOBENZOESÄURE, IOPHENOXINSÄURE;

Farbstoffe aus der Klasse der Azofarbstoffe: beispielsweise P-PHENYLAZOMALEANILIC ACID, 2-(2-PYRIDYLAZO)CHROMOTROPSÄURE, 2,2'-AZOXYDIBENZOESÄURE, 4-(1,8-DIHYDROXY-3,6-DISULFO-2-NAPHTHYLAZO)-SALICYLSÄURE, 3-(2-(2,5-DICHLOROPHENYLAZO)-5-(DIETHYLAMINO)-PHENYLCARBAMOYL)-PROPIONSÄURE, 1-(4-(METHYLTHIO)PHENYL)-3-(4-(PHENYLAZO)PHENYL)HARNSTOFF, 2-HO-5-(4'-HO-BIPHENYL-4-YLAZO)-BENZOESÄURE, PYRIDYL-2-AZOCHROMOTROPSÄURE, SULFONAZO III, BRILLIANT CROCEIN MOO (= ACID RED 73), TROPAEOLIN (=ACID ORANGE 6), CHICAGOBLAU 6B, BORDEAUX R, ERIOCHROMSCHWARZ, ACID RED 151, DISPERSE GELB 7, PONCEAU SS, DIMETHYLAMINO-1-NAPHTHYLAZO-4-METHOXYBENZOLSULFONSÄURE, SUDAN II SÄURE ROT 88, MORDANT BRAUN 1, TRYPANBLAU (=DIREKT BLAU 14), EVANS BLAU, SÄURE ORANGE 8, SUDAN IV, MORDANT BRAUN 33, DIREKTGELB 50, HABA (= 2-(4'-HYDROXYBENZOLAZO)-BENZOESÄURE), CONGO ROT, 8-HYDROXY-7-(4-SULFOLNAPHTHYLAZA)-5-CHINOLINSÄURE, SULFANILAZOCHROMOTROP, NAPHTHOLSCHWARZ, NITROROT, XYLIDINE PONCEAU, ORANGE G, MORDANT GELB 10, BENZOPURPURIN 4B, CROCEIN ORANGE G, METANILGELB, DIREKT ROT 81;

Farbstoffe aus der Klasse der Anthrachinone: beispielsweise ALIZARIN COMPLEXON (=N-(3,4-DIHYDROXY-2-ANTHRACHINONYLMETHYLIMINODIESSIGSÄURE), ALIZARIN, REMAZOL BRILLIANT BLAU R, ALIZARIN GELB GG, ALIZARIN ROT S, ALIZARIN BLUE BLACK B, NUCLEAR FAST REU;

Farbstoffe aus der Klasse der Phtaleine und Sulfophtaleine: beispielsweise BROMKRESOLGRÜN, TETRAIODOPHENOLPHTHALEIN, TETRABROMOPHENOLSULFONPHTHALEIN, BROMCRESOLLIA, THYMOLBLAU, BROMTHYMOLBLAU, KRESOLROT, JODPHENOLBLAU (=TETRAIODOPHENOLSULFONPHTHALEIN), BROMCHLORPHENOLBLAU, TETRABROMPHENOLBLAU, BROMPYROGALLOROT, COOMASSIE BRILLIANTBLAU R250, CHLORPHENOL RED XYLENOLBLAU, SÄURE VIOLET 17;

Di-, Tri-, Tetra-, Penta- und Hexapeptide, die durch Z (= Benzyloxycarbonyl), BOC (N-tert.-Butoxycarbonyl), FMOC (N-9-Fluorenylmethoxycarbonyl), Ac (= Acetyl) bzw. CBZ (=Carbobenzyloxy) substituiert sein können: beispielsweise Z-TYR-TYR-OH, Z-VAL-ALA-OH, Z-ILE-ALA-OH, AC-ALA-ALA-ALA-ALA-OH, Z-VAL-PHE-OH, Z-MET-PHE-OH, DL-LEUCYL-GLYCYL-DL-PHENYLALANIN, Z-PRO-D-LEU-OH, CARBOBENZYLOXY-L-VALYLGLYCYLGLYCIN, Z-LEU-GLY-GLY-OH, Z-ALA-GLY-GLY-OH, Z-ALA-TRP-OH, CARBOBENZYLOXY-L-METHIONYL-L-TRYPTOPHAN, CARBOBENZYLOXY-L-PHENYLALANYL-L-TRYPTOPHANAMID, CARBOBENZYLOXY-L-ISOLEUCYL-L-PHENYLALANINAMID, Z-PHE-MET-OH, Z-VAL-MET-OH, Z-LEU-MET-OH, CARBOBENZYLOXY-L-NORVALYL-L-LEUCIN, H-GLY-LEU-GLY-LEU-OH, Z-ILE-ILE-OH, Z-PHE-ILE-OH, Z-NVA-NVA-OH, N-ALPHA-Z-L-ARGININ HYDROBROMID, N-FORMYL-MET-LEU-PHE, FMOC-ASN-OH, H-PHE-ARG-OH, ARG-ILE, H-PHE-TYR-OH, Z-PHE-TYR, Z-GLU-TYR-OH, TYR-LEU, H-GLU(TYR)-OH, Z-LEU-ALA-OH, Z-TYR-ALA-OH, Z-PHE-ALA-OH, Z-GLY-ALA-OH, Z-GLY-GLY-ALA-OH, H-TYR-PHE-OH, PHE-PHE-PHE, Z-GLU-PHE-OH, Z-GLY-PHE-OH, H-GLU-GLY-PHE-OH, Z-LEU-GLY-OH, H-PHE-ASP-OH, H-ARG-ASP-OH, Z-GLY-ASP-OH, TYR-GLU, H-ARG-GLU-OH, H-GLU-ASP-OH, GLU-GLU, H-GLU-GLU-GLU-OH, Z-ALA-PRO-OH, Z-PHE-PRO-OH, Z-PHE-GLY-OH, H-PHE-GLY-GLY-OH, TYR-GLY-GLY, Z-GLY-GLY-GLY-OH, Z-THR-PHE-OH, Z-TYR-THR-OH, Z-ORN(Z)-OH, Z-HIS-PHE-OH, H-PRO-VAL-ASP-OH, PYR-ASN-GLY-OH, Z-TRP-ALA-OH, H-LEU-TRP-LEU-OH, H-MET-TRP-OH, H-GLU-TRP-OH, H-ARG-TRP-OH, H-LYS-TRP-OH, AC-PHE-TRP-OH, H-TRP-TRP-OH, H-TRP-SER-OH, TRP-PHE, H-TRP-TYR-OH, H-TRP-LEU-OH, H-TRP-GLU-OH, H-ALA-PHE-GLY-OH, H-GLY-GLY-MET-OH, H-GLY-GLY-GLU-OH, ARG-LYS, H-LYS-GLU-OH, H-LYS-TYR-GLU-OH, H-LYS-GLU-GLY-OH, H-LYS-LYS-LYS-OH, Z-LEU-LEU-OH, Z-ALA-LEU-OH, Z-TYR-LEU-OH, Z-PHE-LEU-OH, Z-GLY-GLY-LEU-OH, H-DL-ALA-DL-LEU-GLY-OH, H-MET-PHE-GLY-OH, H-GLU-TYR-GLU-OH, H-MET-ARG-PHE-OH, H-PHE-ILE-OH, H-LEU-TRP-MET-OH, N-FORMYL-MET-PHE-MET, L-GLN-L-GLN-L-GLN, Z-ARG-OH HCL, H-ARG-ARG-OH ACOH, H-ALA-ARG-OH, Z-GLY-HIS-OH, N-ACETYL-5-BENZYLOXY-DL-TRYPTOPHAN, Z-GLY-TRP-OH, H-TRP-ILE-OH, LEU-ARG, Z-GLY-TYR-OH, CARBOBENZYLOXY-BETA-ALANYL-L-ALANIN, Z-GLY-GLY-SER-OH, MET-LEU-PHE, Z-GLY-GLY-PHE-OH, CARBOBENZYLOXY-BETA-ALANYL-L-PHENYLALANIN, Z-GLY-GLY-NLE-OH; H-ARG-ASP-OH, Z-ALA-GLU-OH, Z-GLY-GLY-PRO-OH, Z-ILE-GLY-GLY-OH, Z-BETA-ALA-GLY-GLY-OH, H-GLU-LYS-OH, Z-GLY-GLY-MET-OH, Z-GLY-GLY-ILE-OH, N-CBZ-BETA-ALA-BETA-ALA, N-ACETYL-MET-LEU-PHE, GLU-VAL-PHE, S-DECYLGLUTATHION, S-PROPYLGLUTATHION, S-BUTYLGLUTATHION, S-HEXYLGLUTATHION, S-OCTYLGLUTATHION, S-(P-CHLOROPHENACYL)GLUTATHION, S-(P-AZIDOPHENYLACYL)-GLUTATHION, Z-PRO-LEU-GLY-OH, Z-PRO-LEU-GLY-NH2, N-FORMYL-NLE-LEU-PHE, GLY-PHE-ARG, BOC-PHE-PHE-GLY-OH, H-ASP-GLU-OH, H-TYR-TYR-PHE-OH, H-GAMMA-GLU-TRP-OH, GLUTATHIONSULFONSÄURE, H-ASP-GLN-OH, H-ARG-GLY-ASP-OH, H-LYS-LYS-OH 2 HCL, ARG-SER-ARG, Z-ALA-PRO-TYR-OH, AC-PHE-TYR-OH, H-TYR-TYR-OH; H-ASP-PHE-OH, N-CARBOBENZOXYGLYCYL-DL-PHENYLALANIN, H-GLU-TYR-OH, S-ETHYLGLUTATHION, H-ALA-ALA-ALA-ALA-OH, H-ARG-LYS-OH, Z-VAL-LEU-OH, H-ARG-ARG-OH, N-FORMYL-NLE-LEU-PHE-OH, H-TYR-GLY-GLY-PHE-OH, ALA-GLY-SER-GLU, VAL-GLY-SER-GLU, H-ARG-GLY-ASP-ALA-OH, H-ARG-GLY-ASP-CYS-OH, H-ARG-GLY-ASP-SER-OH, ARG-GLY-GLU-SER, H-ARG-GLY-ASP-VAL-OH, H-GLY-ARG-GLY-ASP-OH, H-LYS-GLY-ASP-SER-OH, AC-ASP-GLU-OH, H-GLY-GLY-TYR-ARG-OH, AC-SER-ASP-LYS-PRO-OH, Z-GLY-GLY-GLY-GLY-GLY-OH, H-ALA-GLY-GLY-GLY-GLY-OH, H-LYS-TYR-LYS-OH, H-D-TYR-TRP-GLY-OH, BOC-ALA-GLY-GLY-OH, GLU-ALA-GLU, LYS-TYR-LYS, SER-ASP-GLY-ARG, AC-TYR-VAL-GLY, BZ-GLY-ALA-PRO, Z-GLY-PRO-LEU, Z-GLY-PRO-LEU GLY, Z-TYR-GLU-OH, H-ALA-ALA-TYR-ALA-ALA-OH, SUC-ALA-ALA-ALA-4M-BETANA [?], H-ARG-TRP-OH SALZ, H-ARG-TYR-OH SALZ, H-ASP-ARG-BETA-NA, H-LYS-PHE-TYR-OH, Z-PRO-LEU-GLY-NHOH, H-GLY-GLY-ARG-ALA-OH, H-GLY-GLY-GLU-ALA-OH, H-GLY-GLY-GLY-GLY-GLY-OH, GLY-GLY-GLY-GLY-GLY-GLY, H-ILE-ARG-OH, H-LYS-THR-TYR-OH, H-LYS-TYR-SER-OH, H-MET-ARG-OH, PHE-GLY-GLY-PHE, H-PHE-TRP-OH, H-TYR-LYS-OH, H-TYR-TRP-OH, VAL-ALA-ALA-PHE, CARBOBENZYLOXYALANYLMETHIONIN, CARBOBENZYLOXYGLYCYLGLYCYLSARCOSIN, CARBOBENZYLOXYISOLEUCYLMETHIONIN, CARBOBENZYLOXYGLYCYLLEUCYLGLYCIN BENZYL ESTER, CARBOBENZYLOXYTRYPTOPHAN BETA-NAPHTHYLAMID, N-BENZYLGLYCYLGLYCYLPHENYLALANIN, CARBOBENZYLOXYNORVALYLTRYPTOPHANAMID, CARBOBENZYLOXYPHENYLALANYLGLYCYLGLYCIN METHYL ESTER, GLU-ALA-GLU-ASN, GLY-ARG-GLY-ASP, GLY-PRO-GLY-GLY, H-MET-GLY-MET-MET-OH, N-ACETYL-BETA-ASP-GLU, N-FORMYL-ALA-GLY-SER-GLU, SUC-ALA-ALA-VAL-OH, BOC-ALA-GLY-GLY-GLY-OH, ORN ORN-ORN, PHE-GLY-PHE-GLY, PRO-THR-PRO-SER, VAL-GLY-ASP-GLU, VAL-THR-LYS-GLY, AC-MET-ALA-SER-OH, AC-PRO-LEU-GLY-OH, BOC-PHE-GLY-OH, Z-TYR-LEU-NH2, H-VAL-VAL-GLY-OH, AC-D-PHE-TYR-OH, Z-HIS-TYR-OH, CARBOBENZYLOXY-L-TRYPTOPHYL-L-PHENYLALANINAMID, CARBOBENZYLOXY-L-TYROSYL-L-ALANINAMID, CARBOBENZYLOXY-L-SERYL-L-PHENYLALANIN, CARBOBENZYLOXY-L-PHENYLALANYLGLYCYLGLYCINAMID, ARBOBENZYLOXYGLYCYLGLYCYL-L-THREONIN, H-GLY-GLY-GLY-GLY-ALA-OH, Z-DL-GLU-OH, Z-HIS-GLY-OH, CARBOBENZYLOXY-L-HISTIDYLGLYCYLGLYCIN, CARBOBENZYLOXY-L-TYROSYL-L-THREONINAMID, Z-TRP-GLY-OH, Z-TYR-VAL-OH, CARBOBENZYLOXY-L-HISTIDYL-L-TYROSINAMID, CARBOBENZYLOXY-S-BENZYL-L-CYSTEINYL-L-LEUCINAMID, N,N'-BIS(CARBOBENZYLOXY)-L-LYSYLGLYCINAMID, CARBOBENZYLOXY-L-TRYPTOPHYL-L-LEUCINAMID, Z-TYR-PHE-OH, CARBOBENZYLOXY-L-ISOLEUCYL-L-TYROSINAMID, Z-TRP-PHE-OH, CARBOBENZYLOXY-L-TRYPTOPHYLGLYCYLGLYCIN METHYL ESTER, CARBOBENZYLOXY-L-SERYL-L-TYROSIN, CARBOBENZYLOXY-L-TYROSYLGLYCIN, Z-GLY-D-TRP-OH, H-TYR-TYR-OH, BOC-D-GLN(XAN)-OH, H-ASP-ASP-ASP-OH, H-ASP-ASP-ASP-ASP-OH, H-ASP-ALA-SER-VAL-OH, H-MET-LEU-PHE-OH ACOH, H-D-TYR-GLU-GLY-OH, H-D-TYR-PHE-GLY-OH ACOH, H-TYR-THR-OH, H-D-TYR-TRP-GLY-OH ACOH, Z-ALA-TYR(BZL)-OH, Z-GLY-HIS-OH, Z-PHE-GLY-GLY-OH, GLYCYLGLYCYLGLYCYLGLYCYLALANIN, TRH-GLY, VAL-THR-CYS-GLY, H-ARG-GLU-OH, H-GLY-GLY-GLY-GLY-GLY-GLY-OH, H-TRP-PHE-OH, H-TYR-GLU-OH, H-ARG-TYR-OH, BOC-GLY-ASP-OH, BOC-PHE-GLY-GLY-OH, FMOC-ALA-ALA-OH, FMOC-ALA-GLY-OH, FMOC-ALA-PRO-OH, FMOC-GLY-GLY-GLY-OH, FMOC-GLY-PHE-OH, FMOC-GLY-PRO-OH, FMOC-GLY-VAL-OH, FOR-VAL-PHE-GLY-OH, H-LEU-LEU-GLY-OH, H-LYS-ARG-OH, H-MET-GLY-GLY-OH, H-MET-HIS-OH, H-PHE-ARG-OH HBR, H-PRO-VAL-GLY-OH, H-TRP-ASP-OH, H-TYR-GLY-GLY-OH, H-TYR-ILE-OH, H-TYR-LEU-OH, Z-ALA-ARG-OH, Z-ALA-ASP-OH, Z-GLU-LEU-OH, Z-GLU-VAL-OH.Z-GLY-GLU-OH, Z-GLY-GLY-TRP-OH, Z-GLY-GLY-TYR-OH, Z-HIS-ALA-OH, Z-HIS-TRP-OH, Z-ILE-SER-OH, Z-DL-LEU-DL-ALA-OH, Z-LEU-D-ALA-OH, Z-LEU-BETA-ALA-OH, Z-LEU-PHE-OH, Z-LEU-PRO-OH, Z-LEU-SER-OH, Z-PHE-PHE-OH, Z-PHE-TRP-OH, Z-PHE-VAL-OH, Z-PRO-BETA-ALA-OH, Z-SER-GLY-OH, Z-TRP-LEU-OH, Z-VAL-VAL-OH, AC-LYS(AC)-D-ALA-D-ALA-OH, AC-LYS(AC)-D-ALA-D-LYS-OH, AC-LYS(AC)-D-GLU-D-ALA-OH, H-D-GLU-D-GLU-OH, H-D-PHE-D-PHE-OH, H-D-VAL-D-VAL-D-VAL-D-VAL-OH, AC-HIS-GLY-HIS-OH, AC-HIS-PRO-PHE-OH, AC-HIS-SER-PHE-OH, AC-TYR-TYR-OH, BOC-GAMMA-GLU-LYS-OH, BOC-GLY-GLY-GLY-LYS-OH, BOC-PHE-GLY-GLY-GLY-OH, FMOC-ILE-PRO-OH, FMOC-NLE-NLE-OH, FMOC-PRO-PRO-OH, FMOC-VAL-PRO-OH, FOR-MET-LEU-GLU-OH, GLUTARYL-GLY-PHE-4M-BETA-NA, GLUTARYL-PHE-BETA-NA, H-ALA-ALA-ALA-ALA-D-GLU-OH, H-ALA-ALA-ALA-ALA-GLU-OH, H-ALA-ALA-ALA-TYR-ALA-OH, H-ALA-ALA-PRO-ALA-ALA-OH, H-ALA-ALA-TYR-ALA-OH, H-ARG-GLN-OH, H-ARG-GLY-GLY-OH, H-ARG-ILE-OH, H-ARG-PHE-ALA-OH, H-ARG-SER-ARG-OH, H-ASN-GLU-OH, H-ASP-ALA-HIS-LYS-OH, H-ASP-ALA-SER-GLY-GLU-OH, H-ASP-ASN-GLN-OH, H-ASP-TRP-OH, H-CIT-PHE-OH, H-D-ARG-PHE-OH, H-D-GLU-GLU-OH, H-D-PHE-SER(BZL)-PHE-OH, H-GAMMA-GLU-GAMMA-GLU-GLN-OH, H-GAMMA-GLU-GAMMA-GLU-GLU-OH, H-GAMMA-GLU-GAMMA-GLU-LYS-OH, H-GLN-TRP-GLU-OH, H-GLU-GLU-ASP-OH, H-GLU-GLU-BETA-NA, H-GLU-GLU-LEU-OH, H-GLU-HIS-GLY-OH, H-GLU-PHE-TYR-OH, H-GLU-PRO-GLU-THR-OH, H-GLU-SER-LEU-PHE-OH, H-GLY-GLY-ARG-ANILIDE, H-GLY-GLY-GLY-BETA-ALA-OH, H-GLY-GLY-LYS-ALA-ALA-OH, H-GLY-GLY-TYR-ALA-OH, H-GLY-LEU-BETA-ALA-OH, H-GLY-LEU-DELTA-AMINOVALERIC ACID, H-GLY-LEU-LEU-GLY-OH, H-GLY-LYS-GLY-OH, H-GLY-LYS-HIS-OH, H-HIS-TRP-LYS-OH, H-ILE-ARG-PRO-OH, H-ILE-GLN-OH, H-LEU-LEU-VAL-PHE-OH, H-LEU-PHE-LEU-OH, H-LYS-ALA-PHE-GLY-OH, H-LYS-ARG-OH, H-LYS-GLY-GLU-OH, H-LYS-GLY-GLY-LYS-OH, H-LYS-GLY-LYS-OH, H-LYS-LEU-LYS-OH, H-LYS-PHE-LYS-OH, H-LYS-PRO-ARG-OH, H-LYS-PRO-ASN-OH, H-LYS-SER-LYS-OH, H-LYS-TYR-THR-OH, H-MET-ASP-GLY-OH, H-MET-GLN-OH, H-MET-LEU-GLY-OH, H-MET-SER-GLY-OH, H-MET-TRP-GLY-OH, H-MET-TYR-LYS-OH, H-ORN-ORN-ORN-OH, H-PHE-ARG-ARG-OH, H-PHE-GLN-GLY-PRO-OH, H-PHE-TRP-NH2, H-PRO-GLU-THR-OH, H-PRO-HIS-GLU-OH, H-PRO-LEU-GLY-GLY-OH, H-PRO-PHE-LYS-OH, H-SER-GLU-GLY-OH, H-SER-TYR-LYS-OH, H-THR-TYR-LYS-OH, H-THR-TYR-SER-LYS-OH, H-TRP-ARG-OH , H-TRP-GLU-TYR-OH, H-TRP-GLY-GLY-TYR-OH, H-TRP-MET-OH, H-TRP-PRO-TYR-OH, H-TYR-ARG-PRO-NH2, H-TYR-ARG-THR-NH2, H-TYR-GLN-OH, H-TYR-GLU-TRP-OH, H-TYR-LYS-TRP-OH, H-TYR-TYR-LEU-NH2, H-TYR-TYR-LEU-OH, H-VAL-ARG-OH; L-BETA-PHENYLLACTYL-GLN-TYR-OH, L-BETA-PHENYLLACTYL-GLY-TYR-OH, L-BETA-PHENYLLACTYL-PHE-TYR-OH, Z-ALA-ALA-LYS-OH, Z-ALA-PRO-GLY-OH, Z-ALA-PRO-LEU-OH, Z-ASP-MET-OH, Z-D-ALA-PHE-OH, Z-D-GLU-TYR-OH, Z-DL-LEU-GLY-OH, Z-GLU-MET-OH, Z-GLY-ALA-ALA-OH, Z-GLY-GLN-OH, Z-GLY-GLY-GLY-GLY-GLY-GLY-OH, Z-GLY-GLY-HIS-OH, Z-GLY-ILE-ALA-OH, Z-GLY-LEU-ALA-OH, Z-GLY-LEU-GLY-OH, Z-GLY-PHE-ALA-OH, Z-GLY-PRO-ALA-OH, Z-GLY-PRO-GLY-GLY-OME, Z-HIS-MET-OH, Z-ILE-HIS-OH, Z-LYS(Z)-ALA-OH, Z-LYS(Z)-GLY-OH, Z-LYS-LEU-OH, Z-PHE-ASP-OH, Z-PHE-GLU-OH, Z-PHE-LEU-ALA-OH, Z-TRP-MET-OH, Z-TRP-VAL-OH, Z-TYR-ILE-OH, Z-VAL-GLY-PHE-OH, Z-VAL-TRP-OH, GLY-GLY-L-PHE-L-MET, L-TRP-TRP, L-TRP-TYR, Z-TYR-GLY-GLY-OH, H-MET-ASP-PHE-NH2, Z-GLY-ARG-OH, Z-ILE-GLU-OH, Z-LEU-D-PHE-OH, Z-PYR-HIS-OH, Z-LEU-GLU-OH, L-ARG-PHE-ALA, L-ARG-ARG, L-ASP-L-ARG, L-ALA-ALA-ALA-L-PRO-L-ALA-ALA, L-ALA-LEU-ALA-GLY, L-ARG-PRO-PRO, N-ACETYL-D-TRP-D-TRP, N-BENZOYL-L-ARG-PHE-ALA, CBZ-DL-PHE-GLY-GLY HYDRAZIDE, CBZ-L-ARG-PHE, CBZ-L-ALPHA-ASP-GLU, CBZ-GLY-GLY-L-ARG, CBZ-L-GLU-TRP, CBZ-D-ALA-L-ALA, CBZ-L-ALA-D-LEU, CBZ-D-LEU-L-ALA, CBZ-L-LEU-D-LEU, CBZ-L-VAL-D-LEU, CBZ-D-VAL-D-LEU, CBZ-L-VAL-D-PHE, GLY-ALA-ALA-D-ALA-L-ALA, ALPHA-L-GLU-L-TRP-L-GLU, GAMMA-L-GLU-GAMMA-L-GLU-L-ALA, L-LYS-L-TRP OH , L-LEU-TRP-MET , L-LEU-TRP-LEU, L-LYS-TYR-LYS, L-PHE-L-ARG, L-PRO-PHE-ARG, L-PHE-D-PHE, L-SER-GLY-ALA-GLY-ALA-GLY, L-TYR-L-MET, L-TRP-GLY-GLY-TYR, D-TRP-D-TRP, L-TYR-TYR, H-THR-ARG-OH, FMOC-PHE-GLY-OH, FMOC-D-CIS-HYP-OH, FMOC-D-ALLO-THR-OH, FMOC-ALLO-THR-OH, FMOC-D-ASP-OH, PYR-TRP-GLY-NH2, H-PHE-PRO-ARG-OH, BOC-GLY-GLY-GLY-GLY-OH, Z-PHE-GLY-OH, FMOC-ALA-ALA-PRO-OH, BOC-GLY-PRO-GLY-OH.

Somit umfassen erfindungsgemäße Ligandenverbindungen mindestens eine therapeutisch und/oder diagnostisch wirksame Substanz und eine Trägermolekül-affine Substanz wie beispielsweise einen Protein-affinen Liganden, wobei zwischen beiden Spacermolekül vorhanden sein kann. Daher weisen erfindungsgemäße Ligandenverbindungen die folgenden allgemeinen Strukturen auf:

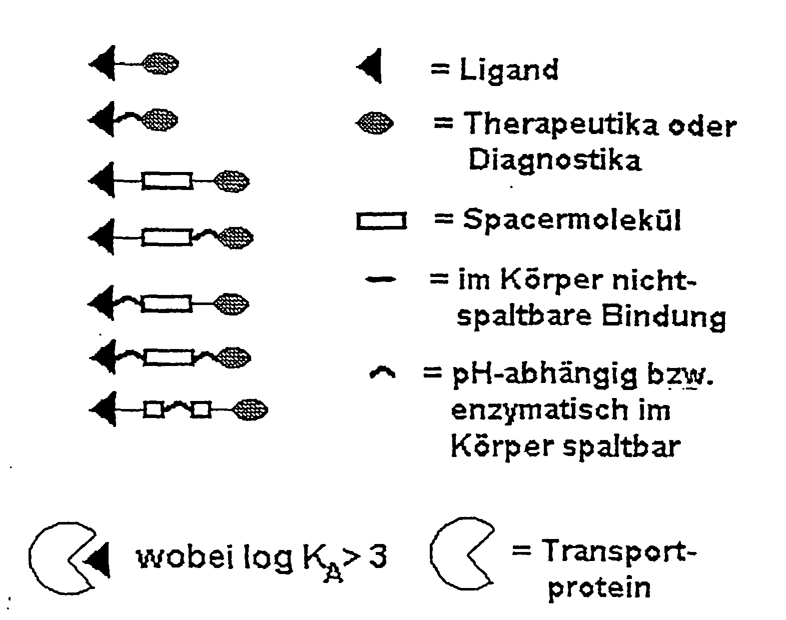

Am Beispiel des Evans Blau und des Bromkresols, die beide eine hohe Affinität zu Albumin besitzen, ist der Aufbau des erfindungsgemäßen Ligandensystems schematisch dargesteilt:
SB = Sollbruchstelle, säurelabil oder enzymatisch spaltbar 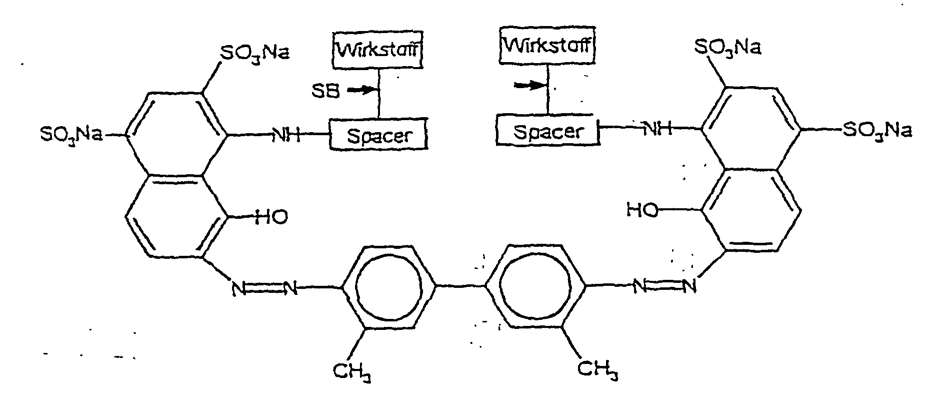
allgemeiner Aufbau eines Protein-affinen Ligandensystems mit Evans Blau 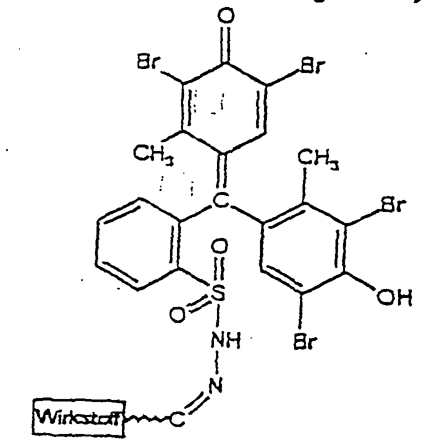 allgemeiner Aufbau eines Protein-affinen Ligandensystems mit Bromkresolgrün

Durch die Bereitstellung der erfindungsgemäßen Ligandenverbindung können überraschenderweise Trägermolekül-affine Substanzen, wie beispielsweise die vorstehend genannten Liganden von Transportproteinen, die mit diesen in der Blutbahn eine starke nicht-kovalente Wechselwirkung eingehen, therapeutisch und/oder diagnostisch genutzt werden, indem therapeutisch und/oder diagnostisch wirksame Substanzen an solche Trägermolekül-affinen Substanzen kovalent gebunden werden. Da solche Ligandensysteme über den Trägermolekül-affinen, beispielsweise Protein-affinen Liganden an das Trägermolekül, z.B. ein Transportprotein, binden, werden die an den Liganden gebundenen Therapeutika bzw. Diagnostika auf diesem Wege zu ihrem Zielort oder ihre Zielzellen bzw. zu einem pathogenen Gewebe wie beispielsweise einem Gewebe, das maligne entartete Zellen aufweist, transportiert. Erfindungsgemaß gewährleist eine säurelabile bzw.enzymatisch spaltbare Bindung zwischen dem Wirkstoff und dem protein-affinen Liganden bzw. Spacer, daß der Wirkstoff am Wirkort intra- oder extrazellulär freigesetzt wird.

Die erfindungsgemäße Ligandenverbindung stellt daher ein neues überlegenes Prodrug-Konzept bereit, da die erfindungsgemäße Ligandenverbindung über die Trägermolekül-affine Substanz, welche eine starke physikalische Wechselwirkung mit dem Trägermolekül, beispielsweise einem Serumprotein wie Albumin eingeht, an den Wirkort transportiert und dort angereichert wird. Die erfindungsgemäße Ligandenverbindung weist außerdem eine hervorragende Löslichkeit im Wirkortmedium auf. Des weiteren hat die erfindungsgemäße Ligandenverbindung den Vorteil, daß zur Herstellung des Konjugats mit dem Trägermolekül letzteres nicht kovalent modifiziert wird, da die Wechselwirkung der Trägermolekül-affinen Substanz mit dem Trägemlolekül physikalischer Natur ist. Bei der Herstellung solcher Konjugate entfallen somit zahlreiche sonst notwendige Arbeitsschritte, was eine erhebliche Zeit- und Materialerspamis und somit eine erhebliche Kostenreduktion mit sich bringt. Dies gilt auch deshalb, da die Ligandenverbindung nicht *ex vivo* mit dem Trägermolekül gekoppelt werden muß, sondern die Konjunktion am Wirkort stattfinden kann.

Eine weitere Ausführungsform der vorliegenden Erfindung betrifft daher ein Addukt bzw. ein Konjugat bzw. einen Komplex eines Trägermoleküls und der wie vorstehend definierten Ligandenverbindung.

Bevorzugte Trägerrnoleküle im erfindungsgemäßen Addukt sind wie oben definiert.

Eine weitere Ausführungsform der vorliegenden Erfindung betrifft ein Verfahren zur Herstellung eines wie oben definierten Addukts, umfassend die Schritte:
(i) Herstellen der vorstehend definierten Ligandenverbindung und
(ii) Inkontaktbringen der Ligandenverbindung mit dem Trägermolekül.

Vorzugsweise umfaßt das Inkontaktbringen den Schritt der oralen Applikation der Ligandenverbindung und/oder den Schritt des Injizierens der Ligandenverbindung in einen Organismus, mehr bevorzugt in die Blutbahn. Diese Vorgehensweise ermöglicht es, wie vorstehend ausgeführt, daß das Trägermolekül, beispielsweise Albumin, nicht isoliert werden muß und darüberhinaus eine weitere Material- und

Zeiterspamis zu erreichen, da ein Syntheseschritt außerhalb des Organismus vermieden wird.

Im Anschluß an die Synthese der-therapeutisch und/oder diagnostisch wirksamen Substanz wird eine injizierbare Arzneimittelzubereitung, enthaltend die therapeutisch oder diagnostisch wirksamen Substanz, in einer geeigneten Trägerflüssigkeit hergestellt. Die therapeutisch und/oder diagnostisch wirksame Substanz liegt in der Regel als Festsubstanz oder als Lösung vor, wobei übliche Träger und/oder pharmazeutische Hilfsstoffe, wie etwa Polysorbate, Glucose, Lactose, Mannose, Mannit, Citronensäure, Tromethamol, Triethanolamin oder Aminoessigsäure beigefügt sein können. Die injizierbare Arzneimittelzubereitung muß so hergestellt werden, daß die therapeutisch oder diagnostisch wirksame Substanz durch das Lösen in der injizierbaren Trägerflüssigkeit nicht deaktiviert, abgespalten oder hydrolysiert wird. Weiterhin muß gewährleistet werden, daß die säurelabile Bindung in der pharmakologisch aktiven Substanz, die beispielsweise eine Ester-, Acetal-, Ketal-, Imin-, Hydrazon, Carboxylhydrazon- oder Sulfonylhydrazon-bindung ist, nicht hydrolysiert wird. In der Regel liegt der pH-Wert im Bereich von pH 5,0 bis 9,0, vorzugsweise von pH 6,0 bis pH 8,0.

Die verwendeten Trägerflüssigkeiten sind annähernd isotonische Puffer, z.B. Phosphat-, Acetat- oder Citratpuffer, wie beispielsweise 0,004 M Natriumphosphat, 0,15 M NaCl - pH 6,0-7,0 oder 0,01 M Natriumacetat, 0,15 M NaCl - pH 5,0-6,5. Die verwendete Trägerflüssigkeit kann auch eine isotonische Natriumchloridlösung sein. Die Puffer können übliche Träger und/oder Hilfsstoffe enthalten, um eine Isotonie zu gewährleisten, wie etwa Polysorbate, Glucose, Lactose, Mannose, Mannitol, Citronensäure, Tromethamol, Triethanolamin oder Aminoessigsäure.

Die Löslichkeit der therapeutisch oder diagnostisch wirksamen Substanz in der injizierbaren Trägerflüssigkeit kann durch pharmazeutische Lösungsmittel, wie beispielsweise Ethanol, Isopropanol, 1,2-Propylenglykol, Glycerol, Macrogole, Polyethylenglykole bzw. Polyethylenoxide oder durch Lösungsvermittler, z.B. Tween, Cremophor oder Polyvinylpyrrolidon, verbessert werden. Zu diesem Zweck wird die therapeutisch oder diagnostisch wirksame Substanz entweder in dem pharmazeutischen Lösungsmittel bzw. Lösungsvermittler gelöst und anschließend mit einem Salzpuffer verdünnt oder eine Trägerflüssigkeit, enthaltend den Salzpuffer und mindestens ein pharmazeutisches Lösungsmittel bzw. Lösungsvermittler, wird zum Lösen der pharmakologisch aktiven Substanz direkt verwendet. Die Konzentration der pharmazeutischen Lösungsmittel bzw. Lösungsvermittler überschreiten dabei nicht die Mengen, die vom Arzneimittelgesetz vorgeschrieben sind.

Der Lösevorgang der therapeutisch oder diagnostisch wirksamen Substanz in der Trägerflüssigkeit ist im allgemeinen nach wenigen Minuten abgeschlossen, so daß eine injizierbare Arzneimittelzubereitung für einen Patienten am Krankenbett zur Verfügung gestellt werden kann.

Gemäß einer weiteren Ausführungsform umfaßt das erfindungsgemäße Verfahren den weiteren Schritt des Bereitstellens des Trägermoleküls, und das Inkontaktbringen der Ligandenverbindung mit dem Trägermolekül erfolgt *ex vivo.* Auf diesem Wege kann, falls notwendig, die Selektivität der Ligandenverbindung für ein

Trägermolekül, beispielsweise einem Transportprotein wie Albumin oder einem Transportprotein, das in geringen Mengen im Blut vorkommt, wie beispielsweise Transcortin, verbessert werden.

Eine weitere Ausführungsform der vorliegenden Erfindung betrifft ein Arzneimittel, enthaltend eine wie oben definierte Ligandenverbindung und/oder ein wie oben definiertes Addukt, und gegebenenfalls mindestens einen pharmazeutisch verträglichen Träger und/oder einen Hilfsstoff und/oder ein Verdünnungsmittel. Das erfindungsgemäße Arzneimittel kann bevorzugt zur Behandlung von Krebskrankheiten, Autoimmunerkrankungen, akuten oder chronisch-entzündlichen Erkrankungen, die durch Viren oder Mikroorganismen, wie beispielsweise Bakterien, und/oder Pilze verursacht werden, verwendet werden.

Noch eine weitere Ausführungsform der vorliegenden Erfindung betrifft einen diagnostischen Kit, enthaltend eine wie oben definierte Ligandenverbindung und/oder eine wie oben definiertes Addukt. Der erfindungsgemäße diagnostische Kit kann bevorzugt zum Nachweis der wie vorstehend definierten Erkrankungen und/oder zum Nachweis von Trägermolekülen und/oder deren Verteilung im Körper verwendet werden.

Die vorstehend beschriebenen therapeutischen und/oder diagnostischen Ligandensysteme stellen Formulierungen von Therapeutika und/oder Diagnostika dar, die aufgrund ihrer Affinität zu bestimmten Trägermolekülen das pharmakokinetische Profil der Therapeutika bzw. Diagnostika entscheidend verändern und verbessem. Nachdem die Ligandenverbindung in eine Körperflüssigkeit gelangt oder auch außerhalb des Körpers mit dem betreffenden Trägermolekül, beispielsweise einem Transportprotein wie Albumin, zusammengebracht wird, bindet sie aufgrund physikalischer Wechselwirkungen an das Trägermolekül, um so als Transportform vorzuliegen, so daß das in der Ligandenverbindung enthaltene Therapeutikum und/oder Diagnostikum zum Zielort transportiert wird und/oder in einer dosierten Form freigesetzt wird.

Die folgenden Beispiele ertäutem die vorliegende Erfindung näher.

### BEISPIELE

Alle Verbindungen wurden durch ¹H- und ¹³C-NMR charakterisert. Für die Säulenchromatogrphie wurde Kieselgel 60, 0,06 mm-0,1 mm verwendet.

### Herstellung der erfindungsgemäßen Trägermolekül-affinen Ligandenverbindung BK-DOX1

Bromkresolgrünsulfonsäurehydrazid wurde mit dem Zytostatikum Doxorubicin zu dem entsprechenden Salfonsäurehydrazon-Derivat umgesetzt (nachstehend mit BK-DOX1 abgekürzt):

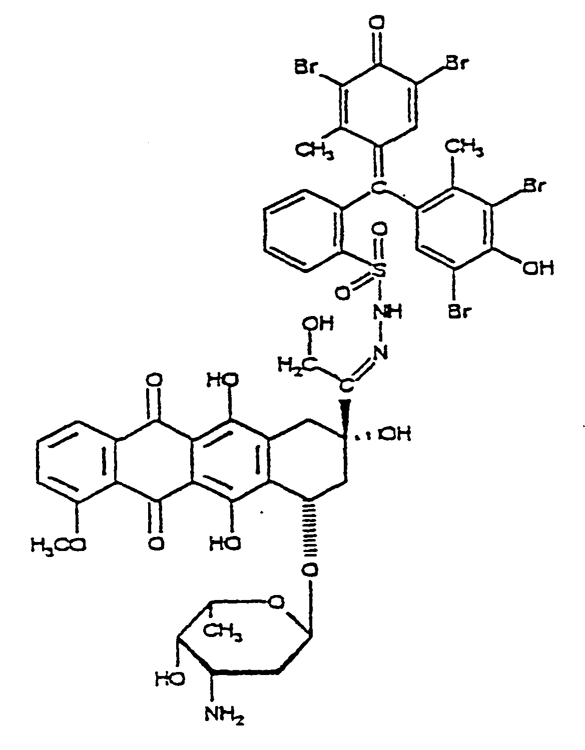

Bromkresolgrünsulfonsäurehydrazid wurde dabei in zwei Schritten - wie nachstehend aufgeführt - hergestellt:
1. Umsetzung von Bromkresolgrünsulfonsäure-Natriumsalz mit N,N'-Dicy- , clohexylcarbodiimid und einem zehnfachen Überschuß an tert.-Butylcarbazat in absolutem Tetrahydrofuran (THF). Das Produkt wurde über Kieselgel isoliert. THF:Hexan = 3:2.
2. Spaltung der BOC-Schutzgruppe mit Trifluoressigsäure und Fällung des Hydrazids mit Ether.

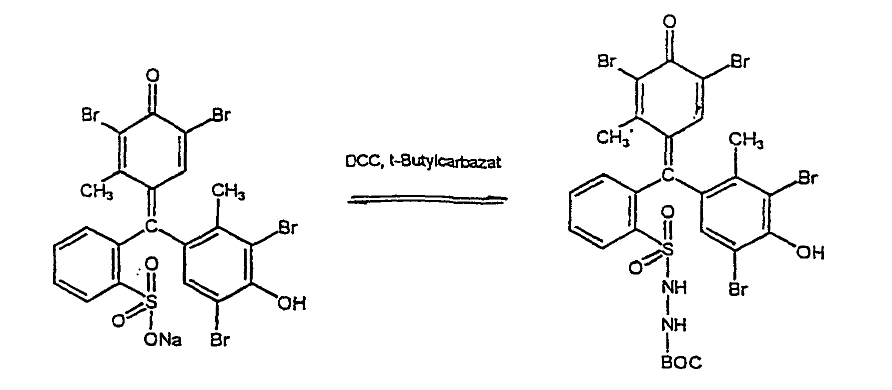

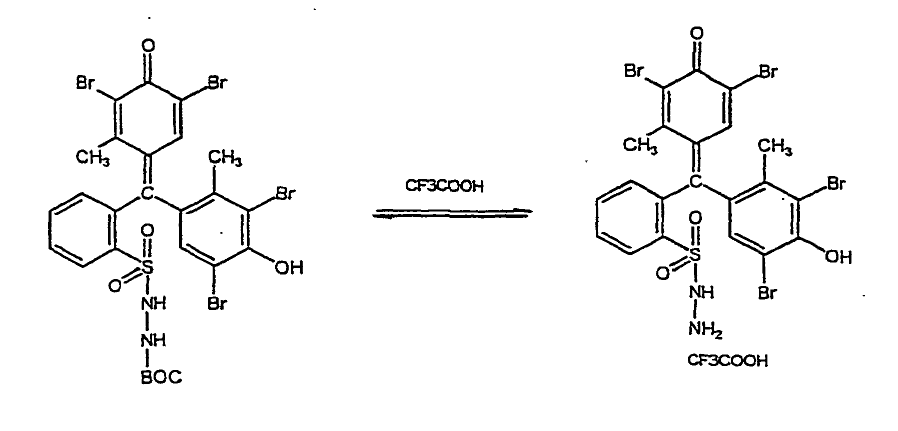

### Synthese von Bromkresolgrünsulfonsäurehydrazid:

4,32 g (6 mmot) Bromkresolgrünsulfonsäure-Natriumsalz und 7,93 g (60 mmol) tert.-Butylcarbazat werden in 50 ml trockenem Tetrahydrofuran gelöst und mit 1,36 g (6,6 mmol) N,N'-Dicyclohexylcarbodümid (DCC), gelöst in 20 ml trockenem Tetrahydrofuran, versetzt. Die Reaktionsmischung wird 16 h lang bei Raumtemperatur gerührt, filtriert und das Lösungsmittel im Vakuum entfernt. Der ölige Rückstand wird säulenchromatographisch gereinigt (Kieselgel; Tetrahydrofuran:Hexan= 3:2). Nach dem Trocknen im Hochvakuum erhält man 300 mg Bromkresolgrünsulfonsäure-tert.-butycarbazatester, Rf-Wert (Tetrahydrofuran : Hexan = 3:2) = 0.35.

200 mg Bromkresolgrünsulfonsäure-tert.-butylcarbazatester werden in 1,0 ml Trifluoressigsäure gelöst und 1 h lang bei Raumtemperatur gerührt. Die Trifluoressigsäure wird im Vakuum entfernt und der Ansatz mit 10 ml trockenem Ether behandelt, so daß man nach dem Abfiltrieren des Feststoffs und Waschen mit n-Hexan 180 mg Bromkresolgrünsulfonsäurehydrazid Trifluoracetatsalz erhält.

### Synthese von BK-DOX-1:

30 mg (0,05 mmol) Doxorubicin und 123 mg (0,15 mmol) Bromkresolgrünsulfonsäurehydrazid Trifluoracetatsalz und 10 µl Trifluoressigsäure werden in 10 ml Ethanol absolut gelöst und 24 Stunden im Dunkeln bei Raumtemperatur gerührt. Anschließend wird auf ~5 ml im Vakuum eingeengt, und das Produkt mit 50 ml wasserfreiem Ethylacetat zur Trübung gebracht und danach bei +5 °C 16 h lang kaltgestellt. Der Niederschlag wird durch Zentrifugation erhalten. Danach wird zweimal mit 6 ml Ethylacetat gewaschen und nach dem Trocknen im Hochvakuum erhält man 45 mg des Produkts. Rf = 0,16 (reversed phase, 50% CH₃CN / 50% K₂HPO₄ pH 7,0, + 2 g/1 Heptansulfonsäure)

### Bindungsverhalten mit Humanserumalbumin

Bromkresolgrün bzw. Bromkresolgrünsulfonsäure weisen Bindungskonstanten für Humanserumalbumin (HSA) von log K_{A} » 10⁷ auf und können selbst nach Inkubationszeiten von nur wenigen Sekunden mit HSA (molares Verhältnis 1:1 bei physiolgischen Konzentrationen) über eine Ausschlußchromatograhie, beispielsweise mit Sephadex® G-25, nicht isoliert werden, sondern eluieren im Gegensatz zu freiem Doxorubicin zusammen mit HSA.

Des weiteren weisen Bromkresolgrün bzw. Bromkresolgrünsulfonsäure keine hohe Affinität zu anderen Plasmaproteinen auf: Wird Bromkresolgrünsulfonsäure mit Transferrin inkubiert, läßt sich Bromkresolgrünsulfonsäure durch Ausschlußchromatograhie mit Sephadex® G-25 fast vollständig zurückgewinnen.

Das Sulfonsäurehydrazon-Derivat BK-DOX1 zeigt bei den oben beschriebenen Inkubationsversuchen ein ähnliches Verhalten wie Bromkresolgrün bzw. Bromkresolgrünsulfonsäure.

Somit zeigt die erfindungsgemäße Trägermolekül-affine Ligandenverbindung BK-DOX1 eine sehr hohe Bindungskonstante für das Transportprotein Humanserumalbumin, die überraschenderweise meherere Zehnerpotenzen größer als diejenige von freiem Doxorubicin ist. Daher ist BK-DOX1 nach Inkontaktbringen mit HSA oder nach Injektion in die Blutbahn fest an dieses Blutprotein gebunden. Da weiterhin BK-DOX1 eine Sulfonylhydrazonbindung als säurelabile Bindung zwischen der Trägermolekül-affinen Substanz und dem Zytostatikum aufweist, kann im sauren Milieu des Tumorgewebes oder in den sauren intrazellulären Kompartimenten der Tumorzelle Doxorubicin als Wirkstoff freigesetzt werden, um dort seine therapeutische Wirksamkeit zu entfalten.

### Herstellung von Doxorubicinderivaten mit Albumin-bindenden Liganden (Crocein Orange, Stearinsäurehydrazid)

### Herstellung des Doxorubicinderivats mit Crocein Orange-Sulfonsäurehydrazid (DOXO-CROC)

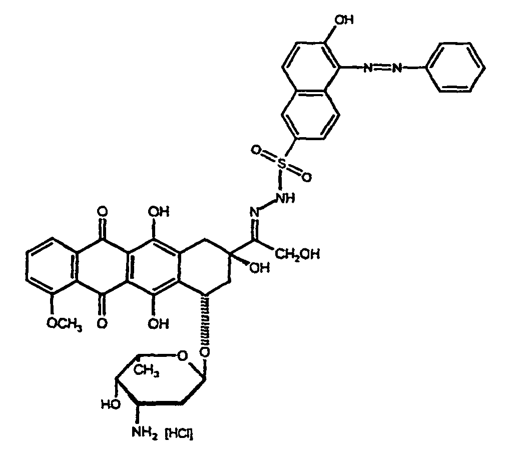

### Herstellung von Crocein Orange G-Säurechlorid

10 g (28,5 mmol) Crocein Orange G (Natriumsalz) und 20 g (96,0 mmol) Phosphorpentachlorid werden als festes Pulver 45 min lang unter Rückfluß bei 150°C erhitzt. Nach dem Abkühlen wird die Reaktionsmischung in 150 ml trockenem Diethylether aufgenommen, filtriert und mit 250 ml trockenem n-Hexan gefällt. Der Ansatz wird bei -20°C 12 h lang gelagert. Anschließend wird der Niederschlag abfiltriert und viermal mit jeweils 60 ml n-Hexan gewaschen. Nach dem Trocknen des Niederschlags im Hochvakuum erhält man 3,2 g als orangenes Pulver. Rf=0,48 (Tetrahydrofuran)

### Herstellung von Crocein Orange Säurehydrazid

3,65 g (10,5 mmol) Crocein Orange-Säurechlorid werden in 10 ml trockenem Tetrahydrofuran gelöst. Unter Rühren werden 2,09 g (15,8 mmol) tert.-Butylcarbazat, gelöst in 15 ml trockenem Tetrahydrofuran, zugegeben. Anschließend werden bei Raumtemperatur 1,46 ml Triethylamin, gelöst in 5 ml trockenem Tetrahydrofuran, zugegeben und 16 h lang gerührt. Der Ansatz wird filtriert und das Lösungsmittel im Vakuum abgezogen. Der ölige Rückstand wird in 50 ml Ethylacetat aufgenommen und einmal mit 100 ml 0,01 N HCI und zweimal mit jeweils 75 ml H₂O extrahiert. Die organische Phase wird über Na₂SO₄ getrocknet und das Lösungsmittel im Vakuum abgezogen. Der Rückstand wird säulenchromatographisch gereinigt (Kieselgel; Ethylacetatin-Hexan = 1: 2). Nach dem Trocknen im Hochvakuum erhält man 2,45 g Croceinorange-Säure-tert.-butylcarbazat als rotes Öl; R_{f} = 0,5 (Ethylacetat:n-Hexan = 1:2).

2,0 g Crocein Orange-Säure-tert.-butylcarbazat werden in 4,0 ml Trifluoressigsäure bei Raumtemperatur gelöst, und der Ansatz 30 min gerührt. Die Trifluoressigsäure wird im Hochvakuum entfernt, so daß man 1,9 g Croceinorangesäurehydrazid (Trifluoracetatsalz) erhält; R_{f} = 0,25 (Ethylactat:n-Hexan =1:2).

200 mg (0,35 mmol) Doxorubicin, 790 mg (1,73 mmol) Croceinorangesäurehydrazid (Trifluoracetatsalz) und 60 µl Trifluoressigsäure werden in 40 ml Methanol absolut gelöst und über Nacht im Dunkeln bei Raumtemperatur gerührt. Anschließend wird der Ansatz filtriert, das Lösungsmittel im Vakuum bis auf etwa 30 ml entfernt und das Produkt mit 80 ml Acetonitril (HPLC-grade) zur Fällung gebracht. Der Ansatz wird 12 h lang bei -20°C gelagert. Danach wird der Niederschlag abzentrifugiert und solange mit Acetonitril gewaschen bis der Überstand farblos ist. Zum Schluß wird einmal mit 10 ml Diethylether gewaschen und nach dem Trocknen im Hochvakuum werden 250 mg des Produkts erhalten. Rf = 0,14 (reversed phase, 50% CH₃CN / 50% K₂HPO₄,pH 7,0 + 2 g/l Heptansulfonsäure)

### Herstellung des Doxorubicinhydrazonderivats mit Crocein Orange und Hexaethylenglykol als Spacer (DOXO-CROC-HEXA)

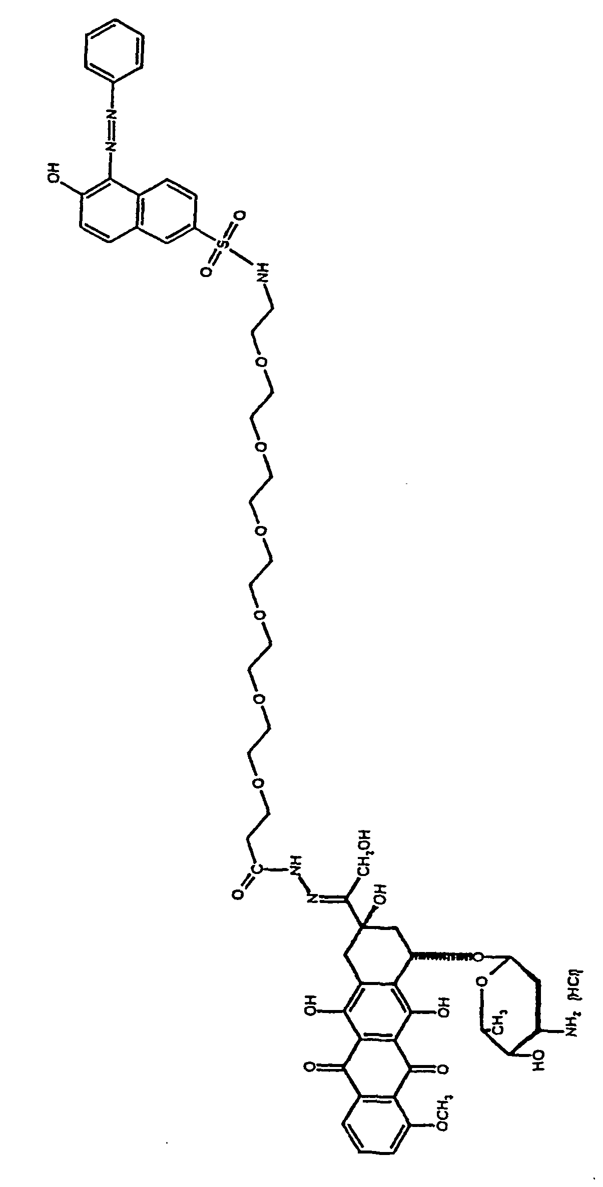

### Synthese des Aminohexaethylenqlykol-t-butylcarbazat-Spacers (4)

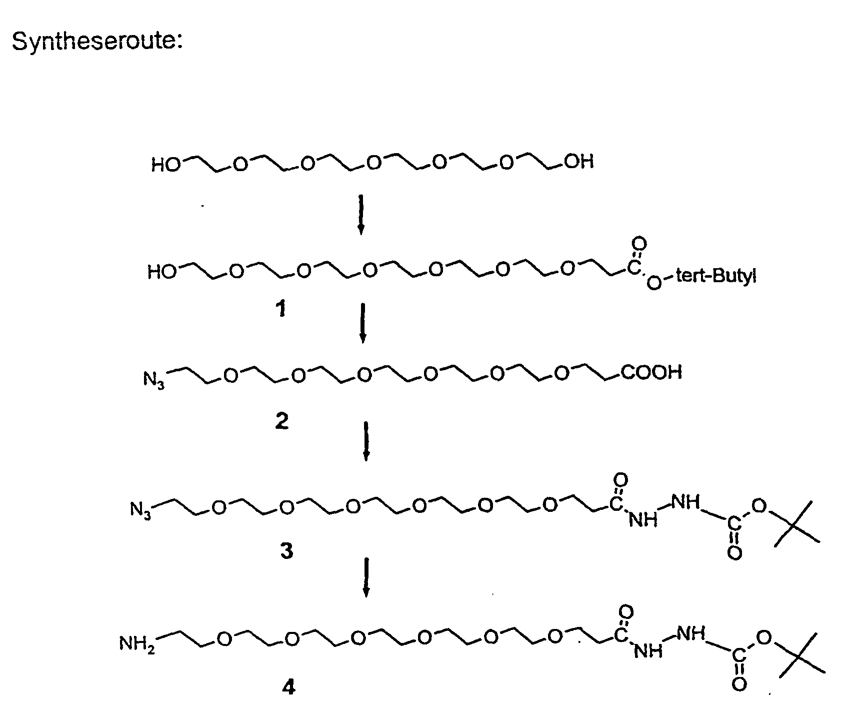

### Synthese der 1. Stufe (1)

51,05g (175,4 mmol) Hexaethylenglykol werden in 90 ml absoluten THF gelöst und mit 211 mg (1,8 mmol) Kalium-tert-butylat versetzt. Unter Rühren werden bei Raumtemperatur innerhalb von 30 min 9,2 ml Acrylsäure-tert.-butylester, gelöst in 15 ml trockenem THF, tropfenweise zugegeben. Der Ansatz wird 16 h lang bei Raumtemperatur gerührt. Die Reaktionsmischung wird unter Rühren mit 2 ml 1 N HCI neutralisiert. Das Lösungsmittel wird im Vakuum entfernt, und der ölige Rückstand wird in 50 ml gesättigter NaCl-Lösung aufgenommen und dreimal mit 50 ml Dichlormethan extrahiert. Die organischen Phasen werden vereint, über Na₂SO₄ getrocknet, filtriert und das Lösungsmittel im Vakuum entfernt. Der ölige Rückstand wird säulenchromatographisch gereinigt (Kieselgel; Ethylacetat:Methanol = 4:1). Nach dem Trocknen im Hochvakuum erhält man 20,25 g als leichtgelbliches Öl. Rf = 0,63 (Ethylacetat: Methanol = 4:1)

### Synthese der 2. Stufe (2)

20,25 g (49,4 mmol) **1** werden in 60 ml absoluten THF gelöst. Unter Rühren werden bei Raumtemperatur zu dieser Lösung 5,73 ml (74,1 mmol) Methansulfonylchlorid und anschließend 10,3 ml Triethylamin (74,1 mmol) zugegeben. Der Ansatz wird 16 h lang bei Raumtemperatur gerührt. Die Reaktionsmischung wird filtriert und das Lösungsmittel im Vakuum entfernt. Nach dem Trocknen im Hochvakuum erhält man 26,98 g als leichtbräunliches Öl, das in 90 ml trockenem Acetonitril gelöst und mit 7,19 g (110,6 mmol) Natriumazid versetzt wird. Die Reaktionsmischung wird 36 h lang unter Rückfluß gerührt. Die Reaktionsmischung wird filtriert und das Lösungsmittel im Vakuum entfernt. Nach dem Trocknen im Hochvakuum erhält man 23,06 g als leichtbräunliches Öl, das in 30 ml Dichlormethan gelöst wird. Unter Rühren werden bei Raumtemperatur innerhalb von 10 min 32,5 ml Trifluoressigsäure tropfenweise zugegeben. Der Ansatz wird 2 h lang gerührt. Das Lösungsmittel und die Trifluoressigsäure werden am Hochvakuum abgezogen. Der ölige Rückstand wird säulenchromatographisch gereinigt (Kieselgel; Ethylacetat:Methanol = 6:1 + 0,5% Essigsäure). Nach dem Trocknen im Hochvakuum erhält man 16,1 g als Öl. Rf= 0,2 (Ethylacetat:Methanol = 6:1 + 0,5% Essigsäure)

### Synthese der 3. Stufe (3)

14,03 g (37,21 mmol) **2** und 5,41 g tert.-Butylcarbazat werden in 420 ml Dichlormethan reinst gelöst und anschließend werden innerhalb von 2 min 6,34 ml Diisopropylcarbodiimid tropfenweise zugegeben. Der Ansatz wird 4 h lang gerührt. Die Reaktionsmischung wird filtriert und das Lösungsmittel im Vakuum abgezogen. Der ölige Rückstand wird säulenchromatographisch gereinigt (Kieselgel; Ethylacetat:Methanol = 12:1). Nach dem Trocknen im Hochvakuum erhält man 10,2 g als Öl. Rf= 0,23 (Ethylacetat : Methanol = 8:1)

### Synthese der 4. Stufe (4)

10,2 g (20,7 mmol) **3** werden in 100 ml absoluten Methanol gelöst und 1,4 g 10%iges Palladium auf Aktivkohle unter Stickstoff portionsweise zugegeben. Anschließend werden 5,4 g (82,9 mmol) Ammoniumformiat, gelöst in 60 ml absolutem Methanol, zugegeben und 12 h lang bei 50°C gerührt. Die Reaktionsmischung wird über Cellite filtriert und das Lösungsmittel im Vakuum entfernt. Der ölige Rückstand wird säulenchromatographisch gereinigt (Kieselgel; Methanol + 1 % Triethylamin). Nach dem Trocknen im Hochvakuum erhält man 8,53 g als Öl. Rf = 0,22 (Methanol +1% Triethylamin)

### Synthese des Hexaethylenglykolhydrazid-Derivats mit Crocein Orange (5):

120 mg (0,24 mmol) **4** und 84 mg (0,24 mmol) Crocein-Orange-Säurechlorid werden in 2 ml trockenem Tetrahydrofuran gelöst und anschließend mit 68 µl Triethylamin versetzt. Der Ansatz wird weitere 2,5 h gerührt. Das Lösungsmittel wird im Vakuum entfernt Nach dem Trocknen im Vakuum erhält man 200 mg als leichtgelbliches Öl, das mit 400 µl Trifluoressigsäure versetzt und 10 min lang gerührt wird. Die Trifluoressigsäure wird im Vakuum entfernt und der ölige Rückstand säulenchromatographisch gereinigt (Kieselgel; Ethylacetat:Methanol = 2:1). Nach dem Trocknen im Hochvakuum erhält man 137 mg des Produkts (Trifluoracetatsalz) als leichtgelbliches Öl; Rf = 0,3 (Ethylacetat/Methanol = 2:1)

### Synthese von DOXO-CROGHEXA:

25 mg (0,04 mmol) Doxorubicin und 104,5 mg (0,15 mmol) Croceinorangehexaethylenglykolhydrazid (Trifluoracetatsalz) (5) werden in 5 ml Methanol absolut gelöst und 24 Stunden lang im Dunkeln bei Raumtemperatur gerührt Anschließend wird das Produkt mit 75 ml Ethylacetat (HPLC-grade) zur Fällung gebracht und 10 Minuten bei +4°C zentrifugiert. Danach wird zwei mal mit 10 ml Ethylacetat gewaschen. Nach dem Trocknen im Hochvakuum erhält man 35 mg des Produkts. Rf = 0,11 (reversed phase, 50% CH₃CN / 50% K₂HPO₄, pH 7,0, + 2 g/l Heptansulfonsäure)

### Herstellung des Doxorubicinhydrazonderivats mit 2,3,5-Triiodobenzoesäure und Hexaethylenglykol als Spacer (DOXO-TIB-HEXA)

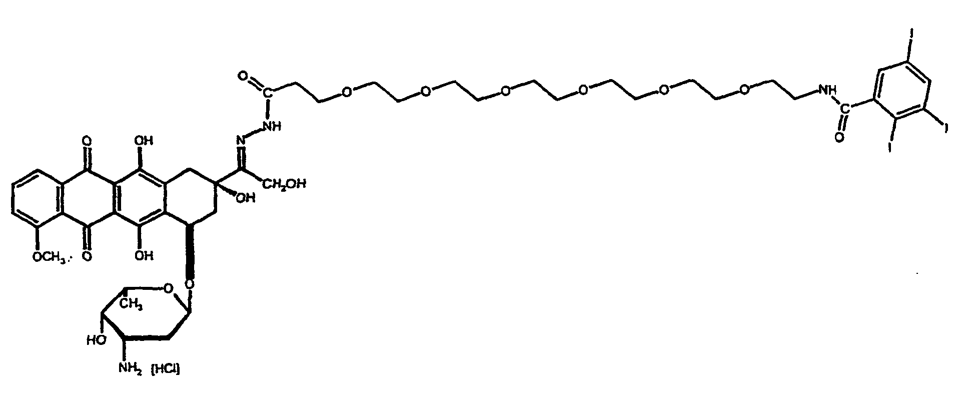

### Synthese des Hexaethylenglykolhydrazid-Derivats mit 2,3,5-Trijodobenzoesäure (6):

124 mg (0,26 mmol) **4,** 132,8 mg (0,26 mmol) 2,3,5-Trijodobenzoesäure und eine Spatelspitze Dimethylaminopyridin werden in 3,0 ml trockenem Tetrahydrofuran gelöst. Der Ansatz wird auf 0°C gekühlt und mit 40 µl Diisopropylcarbodiimid versetzt. Die Reaktionsmischung wird unter Lichtschutz weitere 12 h gerührt. Das Lösungsmittel wird im Vakuum entfernt. Der ölige Rückstand wird säulenchromatographisch gereinigt (Kieselgel; Tetrahydrofuran:Hexan= 8:1). Nach dem Trocknen im Hochvakuum erhält man 195 mg des Produkts (Hexaethylenglykol-tert.butylcarbazatderivat von 2,3,5-Trijodobenzoesäure) als leichtgelbliches Öl; Rf = 0,46 (Tetrahydrofuran:Hexan=8:1). 160 mg des Hexaethylenglykol-tert.butylcarbazatderivats von 2,3,5-Trijodobenzoesäure werden in 500 µl Trifluoressigsäure gelöst und 30 min lang bei Raumtemperatur gerührt. Die Trifluoressigsäure wird im Vakuum entfernt, so daß man 150 mg Hexaethylenglykolhydrazid-Derivat mit 2,3,5-Trijodobenzoesäure als Öl erhält, das direkt weiter verwendet wurde.

### Synthese von DOXO-TIB-HEXA:

30 mg (0,06 mmol) Doxorubicin und 150 mg (0,15 mmol) Trijodobenzoesäurehexaethylenglykolhydrazid (Trifluoracetatsalz) werden in 6 ml Methanol absolut gelöst und 24 Stunden im Dunkeln bei Raumtemperatur gerührt. Anschließend wird das Produkt mit 60 ml Ethylacetat (HPLC-grade) zur Fällung gebracht und 10 Minuten bei +4°C zentrifugiert. Danach wird zweimal mit 6 ml Ethylacetat gewaschen und nach dem Trocknen im Hochvakuum erhält man 42 mg des Produkts. Rf = 0,06 (reversed phase, 50% CH₃CN / 50% K₂HPO₄ pH 7,0, + 2 g/l Heptansulfonsäure)

### Herstellung des Doxorubicinhydrazonderivats mit Stearinsäurehydrazid (DOXO-SSH)

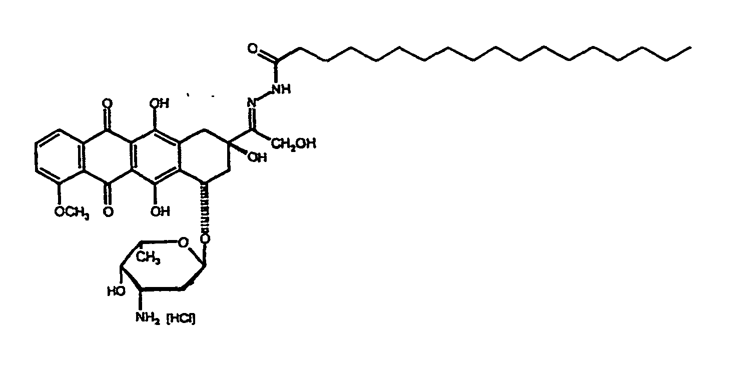

### Herstellung von Stearinsäurehydrazid

*Stearinsäure-tert.-butylcarbazat:* 6,06 g (20 mmol) Stearinsäurechlorid werden in 10 ml trockenem THF gelöst. Bei Raumtemperatur werden unter Rühren innerhalb von 10 min 2,77 ml (20 mmol) Triethylamin und 2,91 g (22 mmol) tert.-Butylcarbazat, gelöst im 10 ml THF, tropfenweise zugegeben und der Ansatz wird 30 min lang gerührt. Die Reaktionsmischung wird filtriert und das Lösungsmittel im Vakuum entfernt. Der ölige Rückstand wird in 50 ml Ethylacetat aufgenommen und zweimal mit 70 ml H₂O extrahiert. Die organische Phase wird über Na₂SO₄ getrocknet, filtriert und das Lösungsmittel im Vakuum entfernt. Der ölige Rückstand wird in 50 ml Ethanol in der Wärme gelöst und 12 h bei +4°C gelagert. Der weiße Niederschlag wird filtriert und zweimal mit jeweils 30 ml Ethanol gewaschen. Nach dem Trocknem im Hochvakuum erhält man 3,6 g Stearinsäure-tert.butylcarbazat; Rf= 0,9 (Ethylacetat/n-Hexan 2/1)

*Stearinsäurehydrazid.* 1,8 g (4,52 mmol) Stearinsäure-tert.butylcarbazat werden in 3 ml Trifluoressigsäure gelöst, 15 min lang gerührt und das Lösungsmittel im Vakuurr entfernt. Der Ansatz wird mit 30 ml trockenem Ether behandelt und der sich bildende Niederschag wird abfiltriert und zweimal mit 50 ml trockenem Diethylether gewaschen. Nach dem Trocknem im Hochvakuum erhält man 1,03 g Stearinsäurehydrazid; Rf= 0,66 (Ethylacetat/ Methanol 1/1)

### Synthese von DOXO-SSH:

50 mg (0,09 mmol) Doxorubicin Hydrochlorid und 42 mg (0,1 mmol) Stearinsäurehydrazid Trifluoracetat werden in 30 ml Methanol (HPLC-grade) gelöst und 18 Stunden im Dunkeln bei Raumtemperatur gerührt. Anschließend wird das Produkt mit 40 ml Acetonitril (HPLC-grade) gefällt. Der Niederschlag wird 10 Minuten bei Raumtemperatur zentrifugiert, danach zweimal mit 30 ml Acetonitril und einmal mit 10 ml Diethylether gewaschen und nach dem Trocknen im Hochvakuum erhält man 60 mg des Produkts; Rf =0,15 (reversed phase, 50% CH₃CN / 50% 20 mM K₂HPO₄ pH 7,0, + 2 g/l Heptansulfonsäure)

### Bindungsverhalten mit Humanserumalbumin

Für die hergestellten Doxorubicinderivate, die alle eine säurelabile Hydrazonbindung als Sollbruchstelle beinhalten, und Doxorubicin wurde durch Gleichgewichtsdialyse (Gleichgewichtsdialyse-Gerät von Spectrum, Inc.,USA; Dialysemembran: cut-off MW 10000) Bindungskonstanten für Humanserumalbumin (HSA der Fa. Dessau, Deutschland) bei pH 7,4 mit Hilfe von Scatchard-Plots bestimmt. Die Ergebnisse sind in der nachstehenden Tabelle 2 zusammengefaßt.

**Tabelle 2**

| **Substanz** | **K**_{**A**} **für HSA** |
|---|---|
| Doxorubicin | 2,9 x 10³ 2,7 x 10³* |
| DOXO-CROC | 6,4 x 10⁶ |
| DOXO-CROC-HEXA | 7,2 x 10⁷ |
| DOXO-TIB-HEXA | 5,5 x 10⁵ |
| DOXO-SSH | 6,1 x 10⁶ |

| | |
|---|---|
| * Literaturwert (Demant et al., Biochemical Pharmacology 55, 27-32, 1998) | |

Die Albumin-bindenden Ligandenverbindungen von Doxorubicin weisen Bindungskonsaten für HSA auf, die um 2-3 Zehnerpotenzen über der Bindungskonstante von Doxorubicin liegen.

Weiterhin können die Albumin-bindenden Ligandenverbindungen von Doxorubicin nach Inkubationszeiten von nur wenigen Sekunden mit HSA oder Blutplasma (molares Verhältnis 1:1, pH 7,0-7,5) über eine Ausschlußchromatograhie, beispielsweise mit Sephadex® G-25, nicht isoliert werden, sondern eluieren im Gegensatz zu freiem Doxorubicin zusammen mit HSA.

Des weiteren weisen die Albumin-bindenden Ligandenverbindungen von Doxorubicin keine hohe Affinität zu anderen Plasmaproteinen auf: Werden sie mit Transferrin inkubiert, lassen sich die albumin-bindenden Ligandenverbindungen von Doxorubicin durch Ausschlußchromatograhie mit Sephadex® G-25 fast vollständig zurückgewinnen.

Somit zeigen die erfindungsgemäßen Trägermolekül-affinen Ligandenverbindungen von Doxorubicin eine sehr hohe Bindungskonstante für das Transportprotein Humanserumalbumin, die überraschenderweise mehrere Zehnerpotenzen größer als diejenige von freiem Doxorubicin ist. Daher weisen die Albumin-bindenden Ligandenverbindungen von Doxorubicin nach Inkontaktbringen mit HSA oder nach Injektion in die Blutbahn eine starke Wechselwirkung mit diesem Blutprotein auf. Da die Albumin-bindenden Ligandenverbindungen von Doxorubicin eine Hydrazonbindung als säurelabile Bindung zwischen der Trägermolekül-affinen Substanz und dem Zytostatikum aufweist, kann im sauren Milieu des Tumorgewebes oder in den sauren intrazellulären Kompartimenten der Tumorzelle Doxorubicin als Wirkstoff freigesetzt werden, um dort seine therapeutische Wirksamkeit zu entfalten.

Die antitumorale Wirksamkeit von DOXO-CROC wurde in einem Melanom-Xenograft-Modell (MV-3) untersucht. Die Daten sind in der nachstehenden Tabelle 3 zusammengefaßt:

**Tabelle 3**

| Substanz | Anzahl der Nacktmäuse | Therapie-schema | Mortalität | Optimaler T/C-wert (%)* |
|---|---|---|---|---|
| NaCl | 8 | 3x (Tag 10,17, 24) | - | |
| DOXO-CROC | 8 | 3x16 mg/kg -i.p. Tween (Tag 10,17, 24) | - | 37 |

| | | | | |
|---|---|---|---|---|
| *Verhältnis der Tumorgrößen der behandelten Gruppe im Vergleich zur Kontrollgruppe | | | | |

Die Therapie mit DOXO-CROC wurde sehr gut vertragen: Die Körpergewichtsabnahme während der Behandlung mit DOXO-CROC war lediglich - 3 %; das Wachstum der subkutanwachsenden Tumoren konnte um über 60% gegenüber der Kontrolle gehemmt werden.

## Patentansprüche

1. Trägermolekül-bindende Ligandenverbindung, umfassend mindestens eine therapeutisch und/oder diagnostisch wirksame Substanz und mindestens eine Trägermolekül-affine Substanz, die eine Assoziationskonstante K_{A} zu einem Trägermolekül von größer 10³ M⁻¹ über eine nicht-kovalente Bindung aufweist, wobei die Verknüpfung zwischen der therapeutisch und/oder diagnostisch wirksamen Substanz und der Trägermolekül-affinen Substanz im Körper pH-abhängig und/oder enzymatisch spaltbar ist, und wobei das Trägermolekül ein Protein, ausgewählt aus der Gruppe der Serumproteine, ist.

2. Ligandenverbindung nach Anspruch 1, wobei die therapeutisch und/oder diagnostisch wirksame Substanz und die Trägermotekül-affine Substanz über ein Spacermolekül miteinander verbunden sind.

3. Ligandenverbindung nach Anspruch 2, wobei das Spacermolekül und/oder die Verknüpfung zwischen der therapeutisch und/oder diagnostisch wirksamen Substanz und dem Spacermolekül und/oder die Verknüpfung zwischen der Trägermolekül-affinen Substanz und dem Spacermolekül im Körper pH-abhängig und/oder enzymatisch spaltbar ist.

4. Ligandenverbindung nach einem der Ansprüche 1 bis 3, wobei die Verknüpfung und/oder das Spacermolekül mindestens eine Peptidbindung enthält.

5. Ligandenverbindung nach Anspruch 4, wobei die Peptidbindung innerhalb einer Peptidsequenz vorliegt, welche mindestens eine Spaltsequenz einer Protease enthält.

6. Ligandenverbindung nach einem der Ansprüche 1 bis 5, wobei die Ver-Knupfung und/oder das Spacermolekul mindestens eine säurelabile Bindung enthält.

7. Ligandenverbindung nach einem der Ansprüche 1 bis 6, wobei das Serumprotein Albumin ist.

8. Ligandenverbindung nach einem der Ansprüche 1 bis 7, wobei die therapeutisch wirksame Substanz ein Zytostatikum, ein Zytokin, ein Immunsuppressivum, ein Antirheumatikum, ein Antiphlogistikum, ein Antibiotikum, ein Analgetikum, ein Virostatikum oder ein AntimyKotikum ist.

9. Ligandenverbindung nach Anspruch 8, wobei das Zytostatikum aus de. Gruppe der Anthrarykline, der N-Nitrosohamstoffe, der Alkylantien, der Purin- oder Pyrimidinantagonisten, der Folsäureantagonisten, der Taxane, der Camptothecine, der Podophyllotoxinderivate, der Vinca-Alkaloide, der Cälicheamicine, der Maytansinoide, der Epithilone oder der *cis*-konfigurierten Platin(II)-Komplexe ausgewählt ist.

10. Ligandenverbindung nach einem der Ansprüche 1 bis 7, wobei die diagnostisch wirksame Substanz ein oder mehrere Radionuklide, ein oder mehrere Radionuklide umfassende Liganden, ein oder mehrere Positronenstrahler, ein oder mehrere NMR-Kontrastmittet, eine oder mehrere fluoreszierende Verbindung(en) oder ein oder mehrere Kontrastmittel im nahen IR-Bereich enthält.

11. Ligandenverbindung nach einem der Ansprüche 1 bis 10, wobei das Spacermolekül einen substituierten oder unsubstituierten, verzweigt- oder unverzweigtkettigen aliphatischen Alkylrest mit 1 bis 20 Kohlenstoffatomen, die teilweise durch Sauerstoff- bzw. Stickstoffatome ersetzt sein können, und/oder mindestens einen substituierten oder unsubstituierten Arylrest umfaßt.

12. Ligandenverbindung nach einem der Ansprüche 1 bis 11, wobei die Träger molekül-affine Substanz aus der Gruppe der Phthalocyanine, der Cumarine, der Flavonoide, der Tetracycline, der Naphthaline, der Aryl- und Heteroarylcarbonsäuren, der langkettigen Fettsäuren, der Pyrrole, der Di- und Tripyrrole, der cyclischen und linearen Tetrapyrrole und ihrer metallorganischen Verbindungen, der mit Halogenatomen (Cl, Br oder 1) 2-5-fach substituierten aromatischen Säurederivaten, der organischen Farbstoffe, der Benzophenone, der Phthal- und Isophthalimide, der Phthal- und lsophthalsäuren, der Chinoline, der Isochinoline, der Anthrachinone, der Anthracene, der Phenanthrene, der Phenanthroline, der Benzylidene, der Di- und Biphenyle, der Indole, der Indane, der Hippursäuren, der Imidazole, der Benzimidazole, der Chinoxaline, der Pyridine, der Pyrimidine, der Piperidine, der Sarcosine, der Oxazole, der Oxadiazole, der Isoxazole, der Pyrazole, der Tri- und Tetrazole, der Benzothiazole, der Triazine, der Morpholine, der Chromene, der tri-, tetra- und pentasubstituierten Ethan- und Propansäuren, der Stilbene und der Tryptophan- und Thyroxin-analogen Verbindungen und den Derivaten davon sowie der Di-, Tri-, Tetra-, Penta- und Hexapeptide ausgewählt ist.

13. Addukt des in den Ansprüchen 1 und 7 definierten Trägermoleküls und der Ligandenverbindung nach einem der Ansprüche 1 bis 12.

14. Arzneimittel, enthaltend die Ligandenverbindung nach einem der Ansprüche 1 bis 12 und/oder das Addukt nach Anspruch 13 und gegebenenfalls mindestens einen pharmazeutisch verträglichen Träger und/oder einen Hilfsstoff und/oder ein Verdünnungsmittel.

15. Arzneimittel nach Anspruch 14 zur Behandlung von Krebskrankheiten, Autoimmunkrankheiten, akuten oder chronisch-entzündlichen Erkrankungen und Erkrankungen, die durch Viren und/oder Mikroorganismen verursacht werden.

16. Diagnostischer Kit, enthaltend die Ligandenverbindung nach einem der Ansprüche 1 bis 12 und/oder das Addukt nach Anspruch 13.

17. Diagnostischer Kit nach Anspruch 16 zum Nachweis von Krebskrankheiten, Autoimmunkrankheiten, akuten oder chronisch-entzündlichen Erkrankungen und Erkrankungen, die durch Viren und/oder Mikroorganismen verursacht werden, und/oder zum Nachweis des Trägermoleküls und/oder dessen Verteilung im Körper.

18. Verfahren zur Herstellung des Addukts nach Anspruch 13, umfassend die Schritte:
(i) Herstellen der Ligandenverbindung nach einem der Ansprüche 1 bis 12 und
(ii) *ex vivo*-Inkontaktbringen der Ligandenverbindung mit dem Trägermolekül.

19. Verwendung der Ligandenverbindung nach einem der Ansprüche 1 bis 12 zur Herstellung eines Arzneimittels für die Behandlung von Krebskrankheiten, Autoimmunkrankheiten, akuten oder chronisch-entzündlichen Erkrankungen und Erkrankungen, die durch Viren und/oder Mikroorganismen verursacht werden, mittels oraler Applikation und/oder Injizierens in die Blutbahn eines Organismus.

## Claims

1. A carrier molecule-binding ligand compound, comprising at least one therapeutically and/or diagnostically effective substance and at least one carrier molecule-affine substance having an association constant K_{A} to a carrier molecule of greater than 10³ M⁻¹ over a non-covalent bond, wherein the linkage between the therapeutically and/or diagnostically effective substance and the carrier molecule-affine substance is cleavable pH-dependently and/or enzymatically in the body, and wherein the carrier moecule is a protein selected from the group of serum proteins.

2. The ligand compound according to claim 1, wherein the therapeutically and/or diagnostically effective substance and the carrier molecule-affine substance are linked with each other over a spacer molecule.

3. The ligand compound according to claim 2, wherein the spacer molecule and/or the linkage between the therapeutically and/or diagnostically effective substance and the spacer molecule and/or the linkage between the carrier molecule-affine substance and the spacer molecule is cleavable pH-dependently and/or enzymatically in the body.

4. The ligand compound according to any of claims 1 to 3, wherein the linkage and/or the spacer molecule contain(s) at least one peptide bond.

5. The ligand compound according to claim 4, wherein the peptide bond is present within a peptide sequence containing at least one cleavage sequence of a protease.

6. The ligand compound according to any of claims 1 to 5, wherein the linkage and/or the spacer molecule contain(s) at least one acid labile bond.

7. The ligand compound according to any of claims 1 to 6, wherein the serum protein is albumin.

8. The ligand compound according to any of claims 1 to 7, wherein the therapeutically effective substance is a cytostatic agent, a cytokine, an immunosuppressant, antirheumatic, antiphlogistic, antibiotic, analgetic, virostatic or antimykotic agent.

9. The ligand compound according to claim 8, wherein the cytostatic agent is selected from the group of anthracyclines, N-nitrosoureas, alkylating agents, purine or pyrimidine antagonists, folic acid antagonists, taxanes, camptothecins, podophyllotoxine derivatives, Vinca alkaloids, calicheamicines, maytansinoids, epithilones or cis-configured platinum(II) complexes.

10. The ligand compound according to any of claims 1 to 7, wherein the diagnostically effective substance contains one or more radionuclide(s), ligands comprising one or more radionuclides, one or more positron emitter(s), one or more NMR contrast agent(s), one or more fluorescent compound(s) or one or more contrast agent(s) in the near IR region.

11. The ligand compound according to any of claims 1 to 10, wherein the spacer molecule comprises a substituted or unsubstituted, branched or unbranched chain aliphatic alkyl residue having from 1 to 20 carbon atoms which may partly be replaced by oxygen or nitrogen atoms, respectively, and/or at least one substituted or unsubstituted aryl residue.

12. The ligand compound according to any of claims 1 to 11, wherein the carrier molecule-affine substance is selected from the group of phthalocyanines, cumarines, flavonoids, tetracyclines, naphthalines, aryl and heteroaryl carboxylic acids, long-chain fatty acids, pyrroles, di- and tripyrroles, cyclic and linear tetrapyrroles and their organometallic compounds, aromatic acid derivatives, 2-5-fold substituted by halogen atoms (Cl, Br or I), organic dyes, benzophenones, phthalic and isophthalic imides, phthalic and isophthalic acids, chinolines, isochinolines, anthrachinones, anthracenes, phenanthrenes, phenanthrolines, benzylidenes, di- and biphenyls, indoles, indanes, hippuric acids, imidazoles, benzimidazoles, chinoxalines, pyridines, pyrimidines, piperidines, sarcosines, oxazoles, oxadiazoles, isoxazoles, pyrazoles, tri- and tetrazoles, benzothiazoles, triazines, morpholines, chromenes, tri-, tetra- and pentasubstituted ethanoic and pentanoic acids, stilbenes, and tryptophane- and thyroxine-analogue compounds and derivatives thereof as well as di-, tri-, tetra-, penta- and hexapeptides.

13. An adduct of the carrier molecule as defined in claims 1 and 7 and the ligand compound according to any of claims 1 to 12.

14. A medicament containing the ligand compound according to any of claims 1 to 12 and/or the adduct according to claim 13 and optionally at least one pharmaceutically acceptable carrier and/or an auxiliary agent and/or a diluent.

15. The medicament according to claim 14 for the treatment of cancerous diseases, autoimmune diseases, acute or chronic-inflammatory diseases and diseases caused by viruses and/or microorganisms.

16. A diagnostic kit containing the ligand compound according to any of claims 1 to 12 and/or the adduct according to claim 13.

17. The diagnostic kit according to claim 16 for the detection of cancerous diseases, autoimmune diseases, acute or chronic-inflammatory diseases and diseases caused by viruses and/or microorganisms, and/or for the detection of the carrier molecule and/or its distribution in the body.

18. A method for producing the adduct according to claim 13, comprising the steps of:
(i) producing the ligand compound according to any of claims 1 to 12 and
(ii) *ex vivo-*contacting the ligand compound with the carrier molecule.

19. Use of the ligand compound according to any of claims 1 to 12 for the manufacture of a medicament for the treatment of cancerous diseases, autoimmune diseases, acute or chronic-inflammatory diseases and diseases caused by viruses and/or microorganisms, by oral application and/or injection into the bloodstream of an organism.

## Revendications

1. Composé de ligands liant une molécule porteuse, comprenant au moins une substance efficace du point de vue thérapeutique et/ou diagnostique et au moins une substance ayant une affinité pour la molécule porteuse, qui présente une constante d'association K_{A} à une molécule porteuse supérieure à 10³ M⁻¹ au moyen d'une liaison non covalente, la liaison entre la substance efficace du point de vue thérapeutique et/ou diagnostique et la substance ayant une affinité pour la molécule porteuse pouvant être clivée d'une manière dépendante du pH et/ou enzymatiquement dans le corps, et la molécule porteuse est une protéine choisie dans l'ensemble des protéines sériques.

2. Composé de ligands selon la revendication 1, dans lequel la substance efficace du point de vue thérapeutique et/ou diagnostique et la substance ayant une affinité pour la molécule porteuse sont liées entre elles au moyen d'une molécule d'espacement.

3. Composé de ligands selon la revendication 2, dans lequel la molécule d'espacement et/ou la liaison entre la substance efficace du point de vue thérapeutique et/ou diagnostique et la molécule d'espacement et/ou la liaison entre la substance ayant une affinité pour la molécule porteuse et la molécule d'espacement peut être clivée d'une manière dépendante du pH et/ou enzymatiquement dans le corps.

4. Composé de ligands selon l'une des revendications 1 à 3, dans lequel la liaison et/ou la molécule d'espacement contient/contiennent au moins une liaison peptidique.

5. Composé de ligands selon la revendication 4, dans lequel la liaison peptidique est présente à l'intérieur d'une séquence peptidique qui contient au moins une séquence de clivage d'une protéase.

6. Composé de ligands selon l'une des revendications 1 à 5, dans lequel la liaison et/ou la molécule d'espacement contient/contiennent au moins une liaison instable vis-à-vis d'un acide.

7. Composé de ligands selon l'une des revendications 1 à 6, dans lequel la protéine sérique est l'albumine.

8. Composé de ligands selon l'une des revendications 1 à 7, dans lequel la substance thérapeutiquement efficace est un cytostatique, une cytokine, un immunosuppresseur, un antirhumatismal, un antiphlogistique, un antibiotique, un analgésique, un virostatique ou un antimycotique.

9. Composé de ligands selon la revendication 8, dans lequel le cytostatique est choisi dans l'ensemble constitué des anthracyclines, des N-nitroso-urées, des alkylants, des antagonistes de la purine ou de la pyrimidine, des antagonistes de l'acide folique, des taxanes, de la camptothécine, des dérivés de la podophyllotoxine, des vincaalcaloïdes, de la calicheamicine, des maytansinoïdes, des épithilones ou des complexes du platine (II) de configuration cis.

10. Composé de ligands selon l'une des revendications 1 à 7, dans lequel la substance efficace du point de vue diagnostique contient un ou plusieurs radionucléides, un ou plusieurs ligands comprenant des radionucléides, un ou plusieurs émetteurs de positrons, un ou plusieurs agents de contraste de RMN, un ou plusieurs composés fluorescents ou un ou plusieurs agents de contraste dans le domaine de l'infrarouge proche.

11. Composé de ligands selon l'une des revendications 1 à 10, dans lequel la molécule d'espacement comprend un radical alkyle aliphatique substitué ou non substitué, à chaîne ramifiée ou non ramifiée, ayant de 1 à 20 atomes de carbone, qui peuvent être partiellement remplacés par des atomes d'oxygène ou d'azote et/ou au moins un radical aryle substitué ou non substitué.

12. Composé de ligands selon l'une des revendications 1 à 11, dans lequel la substance ayant une affinité pour la molécule porteuse est choisie dans l'ensemble constitué des phtalocyanines, des coumarines, des flavonoïdes, des tétracyclines, des naphtalines, des acides aryl- et hétéroarylcarboxyliques, des acides gras à chaîne longue, des pyrroles, des di- et tripyrroles, des tétrapyrroles cycliques et linéaires et de leurs composés organométalliques, des dérivés d'acides aromatiques substitués de 2 à 5 fois par des atomes d'halogène (Cl, Br ou I), des colorants organiques, des benzophénones, des phtalimides et des isophtalimides, des acides phtalique et isophtalique, des quinoléines, des isoquinoléines, des anthraquinones, des anthracènes, des phénanthrènes, des phénanthrolines, des benzylidènes, des di- et biphényle, des indoles, des indanes, des acides hippuriques, des imidazoles, des benzimidazoles, des quinoxalines, de la pyridine, de la pyrimidine, de la pipéridine, de la sarcosine, des oxazoles, des oxadiazoles, des isoxazoles, des pyrazoles, des tri- et tétrazoles, des benzothiazoles, de la triazine, de la morpholine, des chromènes, des acides éthanoiques et propanoïques tri-, tétra- et penta-substitués, des stilbènes et des composés analogues du tryptophane et de la thyroxine et des dérivés de ceux-ci ainsi que des di-, tri-, tétra-, penta- et hexapeptides.

13. Produit d'addition de la molécule porteuse définie dans les revendications 1 et 7 et du composé de ligands selon l'une des revendications 1 à 12.

14. Médicament contenant le composé de ligands selon l'une des revendications 1 à 12 et/ou le produit d'addition selon la revendication 13 et éventuellement au moins un véhicule et/ou un adjuvant et/ou un diluant pharmaceutiquement acceptables.

15. Médicament selon la revendication 14 pour le traitement de maladies liées au cancer, de maladies auto-immunes, de maladies inflammatoires aiguës ou chroniques et de maladies qui sont provoquées par des virus et/ou des micro-organismes.

16. Kit de diagnostic contenant le composé de ligands selon l'une des revendications 1 à 12 et/ou le produit d'addition selon la revendication 13.

17. Kit de diagnostic selon la revendication 16 pour la détection de maladies liées au cancer, de maladies auto-immunes, de maladies inflammatoires aiguës ou chroniques et de maladies qui sont provoquées par des virus et/ou des micro-organismes et/ou pour la détection de la molécule porteuse et/ou de sa distribution dans le corps.

18. Procédé de préparation du produit d'addition selon la revendication 13, comprenant les étapes consistant à :
(i) préparer le composé de ligands selon l'une des revendications 1 à 12 et
(ii) amener le composé de ligands au contact de la molécule porteuse *ex vivo.*

19. Utilisation du composé de ligands selon l'une des revendications 1 à 12 pour la préparation d'un médicament pour le traitement de maladies liées au cancer, de maladies auto-immunes, de maladies inflammatoires aiguës ou chroniques et de maladies qui sont provoquées par des virus et/ou des micro-organismes par administration orale et/ou injection dans le circuit sanguin d'un organisme.
